(19) Europäisches Patentamt
European Patent Office
Office européen des brevets

(11) **EP 1 064 398 B1**

(12) **EUROPEAN PATENT SPECIFICATION**

(45) Date of publication and mention of the grant of the patent:
**06.10.2010 Bulletin 2010/40**

(21) Application number: **99909679.5**

(22) Date of filing: **26.02.1999**

(51) Int Cl.:
*C12Q 1/68* (2006.01)   *C07H 21/04* (2006.01)

(86) International application number:
**PCT/US1999/004376**

(87) International publication number:
**WO 1999/043858 (02.09.1999 Gazette 1999/35)**

(54) **TWO-DIMENSIONAL LINKAGE STUDY TECHNIQUES**

TECHNIKEN ZUR ZWEIDIMENSIONALEN ABHÄNGIGKEITSANALYSE

TECHNIQUES D'ETUDE DE LIAISONS BIDIMENSIONNELLES

(84) Designated Contracting States:
**AT BE CH DE DK ES FI FR GB GR IE IT LI LU MC NL PT SE**

(30) Priority: **26.02.1998 US 76102 P**
**27.02.1998 US 76182 P**
**27.05.1998 US 86947 P**
**07.11.1998 US 107673 P**

(43) Date of publication of application:
**03.01.2001 Bulletin 2001/01**

(73) Proprietors:
• **McGinnis, Ralph Evan**
**Sawbridgeworth, Herts CM21 9PR (GB)**
• **McGinnis, Robert Owen**
**Bozeman, MT 59715 (US)**

(72) Inventors:
• **McGinnis, Ralph Evan**
**Sawbridgeworth, Herts CM21 9PR (GB)**
• **McGinnis, Robert Owen**
**Bozeman, MT 59715 (US)**

(74) Representative: **Obenland, Sigrid**
**Pegnitzstrasse 7**
**80638 München (DE)**

(56) References cited:
**EP-A- 0 785 280     EP-A- 0 892 068**
**WO-A-00/28080**

• **LINDLÖF M ET AL: "Linked polymorphic DNA markers in the prediction of X-linked muscular dystrophy." ANNALS OF HUMAN GENETICS. OCT 1987, vol. 51 ( Pt 4), October 1987 (1987-10), pages 317-328, XP009043751 ISSN: 0003-4800**

• **RAMSAY MICHELE ET AL: "Haplotype analysis to determine the position of a mutation among closely linked DNA markers" HUMAN MOLECULAR GENETICS, vol. 2, no. 7, 1993, pages 1007-1014, XP009043773 ISSN: 0964-6906**
• **DEVLIN B ET AL: "A comparison of linkage disequilibrium measures for fine-scale mapping" GENOMICS, vol. 29, no. 2, 1995, pages 311-322, XP001205308 ISSN: 0888-7543**
• **COLLINS A ET AL: "Mapping a disease locus by allelic association" PROCEEDINGS OF THE NATIONAL ACADEMY OF SCIENCES OF THE UNITED STATES OF AMERICA, vol. 95, no. 4, 17 February 1998 (1998-02-17), pages 1741-1745, XP001205310 ISSN: 0027-8424**
• **MCGINNIS R E: "Hidden linkage: a comparison of the affected sib pair (ASP) test and transmission/disequilibrium test (TDT)." ANNALS OF HUMAN GENETICS. MAR 1998, vol. 62 ( Pt 2), March 1998 (1998-03), pages 159-179, XP009043750 ISSN: 0003-4800**
• **KRUGLYAK et al., "Linkage Thresholds for Two-Stage Genome Scans", AM. J. HUM GENET., 1998, Vol. 62, pages 994-996, XP002920340**
• **KRUGLYAK L., "The Use of a Genetic Map of Biallelic Markers in Linkage Studies", NATURE GENETICS, September 1997, Vol. 17, pages 21-24, XP002920343**
• **KRUGLYAK et al., "Parametric and Nonparametric Linkage Analysis: A Unified Multipoint Approach", AM. J. HUM. GENET., 1996, Vol. 58, pages 1347-1363, XP002920344**
• **KRUGLYAK et al., "Complete Multipiont Sib-Pair Analysis of Qualitative and Quantitative Traits", AM. J. HUM. GENET., 1995, Vol. 57, pages 439-454, XP002920345**

- CHEE M ET AL.: SCIENCE, vol. 274, 1996, pages 610-614, XP002022508
- YESRSHOW ET AL: PROC.NATL.ACAD.SCI., vol. 93, 1996, pages 4913-4918, XP000196978
- GUS Z ET AL: NUCLEIC ACIDS RESEARCH, vol. 22, no. 24, 1994, pages 5456-5465, XP008018942
- DROBYSHEV A. ET AL: "Sequence analysis by hybridization with oligonucleotide microchip: Identification of beta-thalassemia mutations" GENE, vol. 188, no. 1, 1997, pages 45-52, XP004115664

**Description**

Technical Field

Versions of the present invention are in the field of molecular biology, some versions are specifically in the area of finding the chromosomal location of genes that cause genetic characteristics such as human disease.

Background

Introduction

Conventional linkage study techniques have limited power to localize trait causing genes ( trait causing polymorphisms ) of modest effect, such as many human disease polymorphisms. The two-dimensional linkage study techniques of this application are powerful new techniques for localizing genes (polymorphisms) especially of modest effect.

Chromosomes, heredity, genes, markers and alleles

Chromosomes are large molecules that carry the information for the inheritance of physical (genetic) characteristics or traits. In human beings for example, parents pass a copy of half of their chromosomes to their offspring during reproduction. By doing this, each parent passes some of his or her physical characteristics to his or her offspring. Any chromosome of a living creature is made of a large string-like molecule of DNA Chromosomes are essentially very long strings of DNA Genes are small pieces of a chromosome that cause or determine inherited genetic characteristics. (In this application, the term gene means a polymorphism that determines a genetic characteristic; the term does not mean an entire gene structure with a promoter region, introns, etc..) Markers are any segment of DNA on a chromosome which can be identified and whose chromosomal location is known (at least to some extent). Markers are like milestones along the very long string-like molecule of DNA which makes up a chromosome. Both a gene and a marker can come in different forms on different chromosomes. These different forms are known as different alleles and when a gene or marker comes in different forms it is said to be "polymorphic". For example, a bi-allelic marker comes in two (bi) different forms.

Linkage

If a gene allele and a marker allele occur as part of the genetic makeup of individuals more frequently than would be expected on the basis of chance, then it is possible to infer that the gene and the marker are linked. If a gene allele and a marker allele are inherited together more frequently than would be expected if the gene and the marker were on different chromosomes, then it is possible to infer that the gene and the marker are linked. Linkage of a gene and a marker usually occurs because the gene and the marker are close together on a chromosome. There are different degrees of linkage. Establishing linkage, especially strong linkage, between a gene and a marker can be very valuable. This is especially true if the precise location and other characteristics of the gene are not known. By establishing linkage, especially strong linkage, between a known marker and an unknown gene it is possible to locate the gene near to the chromosomal location of the known marker. This can be very valuable if the gene is an important gene, such as a disease causing gene, and can help cure the disease.

Linkage Studies

Linkage studies are a method of establishing linkage between a marker and a gene or genes. Linkage studies are used to statistically correlate the occurrence of a genetic characteristic such as a disease (caused by a gene or genes) with a marker on a chromosome. One way this is done is by statistically correlating a specific allele of a marker with a genetic characteristic for a set of individuals by showing that individuals with the characteristic inherit the marker allele more often than individuals without the characteristic. The set of individuals is usually referred to as a sample of individuals. An example of a sample of individuals are people with a disease and similar people (matched controls) without the disease. Another example of a sample of individuals is a group of people, some of whom have the same disease; each of the people in the group being related to one or more of the other people in the group (i.e. families, sibships, pedigrees). The presence or absence of a marker allele in the chromosomal DNA of each individual is usually determined by genotype data at the marker for each individual.

There are different types of linkage study techniques, using different types of samples and different statistical measures of the correlation of a marker and a genetic characteristic. One example of a type of linkage study technique is the affected sib pair (ASP) test. Another example is the transmission disequilibrium test (TDT), which is an association based linkage test. This is a dynamic, changing area within the field of human genetics.

Linkage Studies and the "Scanning" of Chromosomal Regions

There are significant advantages in using several markers simultaneously to perform a linkage study with a genetic characteristic and a sample of individuals, especially when the relative positions of the markers on a chromosome are known. Such a linkage study allows searching for statistical evidence of linkage between markers in one or more regions of a chromosome or chromosomes and the gene or genes that determine the genetic characteristic. The results of the study for each marker can then be compared with the results for other markers, knowing the relative chromosomal positions of all the markers in the study. In this way, regions of a chromosome or even whole chromosomes can be "scanned" for evidence of linkage to a gene or genes causing a genetic characteristic. The relative positions of markers on chromosomes of a species of creatures is given by various kinds of chromosomal maps for the species. (There are several different kinds of marker maps, i.e. physical maps, genetic maps, radiation hybrid maps, etc.)

Sets of Markers for Linkage Studies and "Scanning" Chromosomes

An appropriate set of markers from a region of a chromosome can be chosen so that the region can be "scanned" for evidence of linkage of markers in the region to a gene or genes that cause a genetic characteristic. As explained above, this scanning is done by using the markers in linkage studies. Strong positive evidence for linkage of the markers (from the scanned chromosomal region) to a gene or genes responsible for a characteristic or trait is strong evidence that a trait-causing gene or genes is located within the chromosomal region.

Conventional Techniques for Choosing Sets of Markers to Scan Chromosomes with Linkage Studies Conventional techniques choose sets of markers to scan a chromosomal region by choosing markers according to each marker's chromosomal location within the region. In a set of microsatellite markers described in 1994 for use in linkage studies, the markers were approximately evenly spaced, with average spacing between markers being 13 centiMorgans. The markers were distributed approximately evenly across the entire human genome (all human chromosomes) and were also selected because genotype data at the markers for individuals could be obtained by a semi-automated method.[1] A recent (1998) linkage study of the disease schizophrenia used a set of 310 microsatellite markers distributed approximately evenly across the entire human genome with average spacing of 11 centiMorgans between markers.[2] In a recent (1998) simulation of linkage studies to defend the practice of two-stage genome scanning, markers were spaced evenly every 10 cM(centimorgans) in an initial, sparser, first stage scan and evenly every 1 cM in a followup, denser, second stage scan.[3] Following up positive linkage study results from chromosomal regions in a sparse, first stage scan with a second, denser scan that focuses on studying the regions with positive first-stage results is a common technique. In these conventional studies, as is common, markers were chosen to be about evenly spaced across the chromosomal regions studied. In this manner, as is conventional, a one dimensional structure such as an entire genome, a chromosome or a region of a chromosome is "covered" by markers in order to scan the entire genome, chromosome or chromosomal region with a linkage study. (These conventional techniques[1, 2, 3] are not admitted to be prior art by their mention in this background.) ( There is a possibly confusing, double meaning, of the term "marker map". It should be noted that a set of markers distributed along a chromosomal region, chromosome, or genome for linkage studies is also sometimes referred to as a "marker map" for use in chromosomal scanning by linkage studies. In addition, chromosomal or genetic maps of markers are also referred to as "marker maps".)

**Conventional Techniques for Choosing Sets of Markers to Scan Chromosomal Regions are Essentially One Dimensional**

Because DNA is a stringlike molecule, a chromosomal region(s), chromosome(s) and genome are essentially one dimensional in terms of the chromosomal location of markers and genes. As has been stated, conventional linkage study techniques scan a chromosomal region(s), chromosome(s) or genome by using markers distributed approximately evenly along the length of the chromosomal region(s), chromosome(s) or genome respectively. These conventional techniques focus primarily on the chromosomal location of markers used in a scan. These conventional techniques have an essentially one dimensional perspective.

Population Frequency of Marker Alleles and Gene Alleles

As described, chromosomal location of each marker is an important and unique characteristic of each marker and marker allele. Another characteristic of each polymorphic marker and each of the marker's alleles is the population frequency of each marker allele. A population is a group (usually a large group) of individuals. A population frequency of a particular marker allele is the proportion of individual chromosomes in a population in which the particular marker occurs as the particular marker allele. For any bi-allelic marker, knowing the least common allele frequency of the marker establishes both of the allele frequencies of the marker. This is because the two allele frequencies of a bi-allelic marker sum to 1. Each gene allele also has a population allele frequency or allele frequency for short. Thus, each gene allele has a particular chromosomal location and allele frequency (for a particular population). In the case of an unknown gene, the gene's chromosomal location and allele frequencies are not specifically known.

Marker Allele Population Frequency in Conventional Linkage Study Scans

It is important to note that little attention was paid to the population allele frequencies of the markers used in the conventional linkage scans cited above. In the two studies cited above under conventional scanning techniques[1,2], marker allele frequency is referred to only peripherally as average marker heterozygosity, which is related to average marker allele frequency and the number of alleles (2, 3, 4, 5, etc.) at each marker. In the simulated scan cited above[3], *the markers are stipulated to have four alleles that all have exactly same allele frequency of 0.25* (heterozygosity 0.75). ***It is important to note that while the chromosomal location of the markers in all these conventional scans was systematically varied over the entire genome (all the human chromosomes), nothing was said about systematically varying the allele frequencies of the markers in any of the scans.*** This is typical of conventional linkage study scans of genomes, chromosomes and chromosomal regions.

A Conventional View Of Bi-allelic Markers And Linkage Studies

We cite here a well known reference that discusses the conventional view of bi-allelic marker usefulness in linkage scans of chromosomes. In 1997 Kruglyak carried out computer simulations of the "information content" of markers that are part of various different marker maps.[4] For bi-allelic markers his results showed that the optimum allele frequencies for bi-allelic markers used in linkage studies is 0.5/0.5 in order to achieve the greatest information content. However, allele frequency patterns other than the optimum 0.5/0.5 for bi-allelic markers gave acceptable levels of information content depending on the density of the marker map (or set of markers) chosen for the linkage study.

There are some important observations regarding this reference.[4] First, ***there is no advantage noted in this reference for choosing bi-allelic markers so that the set of chosen markers (or marker map) used for linkage studies is such that the markers systematically vary in allele frequency.*** Thus, just as in the recent conventional linkage study scans cited above, there is no definite thought to using markers of systematically varying allele frequencies. The greatest information content is given by bi-allelic markers with allele frequencies close to the optimum of 0.5/0.5. Given the density of reasonably polymorphic SNPs predicted in this reference, at least one every 1 kb or 1,000 per cM, it is probable that even for quite dense maps, there will be so many acceptable SNPs available, that all of the SNPs in an appropriate marker map could have the optimum allele frequencies of approximately 0.5/0.5. Secondly, bi-allelic markers with lower least common allele frequencies, less than 0.3(0.7/0.3) or 0.2(0.8/0.2), are viewed unfavorably for linkage studies in this reference. Thirdly, the early version of the criterion of "information content" of markers used in this reference was based on sib pair analysis and the later, current version of the criterion, does not depend on any particular test for linkage.[5,6] **Thus, the criterion of information content in this reference, has never specifically employed the TDT (transmission disequilibrium test) or any association based test, whereas the two-dimensional linkage study techniques of this application are based on a completely different perspective of using association based tests.** (This reference[4] is not admitted to be prior art with respect to the present invention by it's mention in this background.)

Increased Power of the TDT (transmission disequilibrium test)

Characteristics of a new type of linkage test, the TDT (transmission disequilibrium test), were described in 1993. The inventor, R.E.McGinnis, was one of the authors of this reference.[7] In 1996, Risch and Merikangas argued that conventional linkage analysis has limited power to detect genes of modest effect. And Risch and Merikangas attempted to illustrate the increased power of association based linkage tests such as the TDT over other types of conventional linkage tests.[8] However, Risch and Merikangas' analysis was criticized by Muller-Myhsok and Abel as being based on the optimal assumption that the analyzed allele was the disease allele itself. They said that association studies such as the TDT can be quite strong when there is a high probablility that the allele studied is the causal allele, but in other cases the number of families necessary for a TDT study increases dramatically as p differs from m, even when $\delta = \delta_{max}$, and also as $\delta$ decreases. Muller-Myhsok and Abel concluded that researchers should be aware that the power of association studies such as the TDT can be greatly diminished as soon as the ratio m/p departs from unity and the linkage disequilibrium becomes weaker, as is the more common, less optimal situation.[9] In their response to Muller-Myshok and Abels' letter, Risch and Merikangas essentially agreed with the logic of Muller-Myshok and Abels' criticism. Risch and Merikangas stated that to a large extent, the expectation with respect to linkage disequilibrium across the genome is uncharted territory.[10] (None of the references in this paragraph [7,8,9,10] is admitted to being prior art with respect to the present invention by their mention in this background.)

## More Detailed Studies of the Power of the TDT

The inventor, R.E.McGinnis, has done extensive investigations on the power of the TDT. His observations and calculations of the increased power of the TDT in many situations have been published.[11] In this paper a general framework for determining the power of the TDT in many different situations is presented. The analysis of Risch and Merikangas [8] and others is shown by the inventor to be a special case of his general framework. His observations and calculations published in this paper have shown that the TDT has increased power in more common, less optimal situations as well as the less common, optimal situation cited by Muller-Myshok and Abel [9]. As opposed to the observation of Muller-Myhsok and Abel, the inventor's calculations indicate that association tests such as the TDT have increased power in typical situations such as when the ratio m/p departs significantly from unity and, or the linkage disequilibrium between the analyzed (marker) allele and disease polymorphism is only half its maximum possible value. Muller-Myshok and Abel emphasized the weakness of TDT power when the m/p ratio departs from unity and $\delta$ is not close to $\delta_{max}$.
The inventor arrived at these conclusions independently and did not derive them from others.

### *A Major Conclusion Drawn by the Inventor about the TDT and Linkage Studies: Using Bi-allelic Markers of Systematically Varying Allele Frequencies Increases the Power of Linkage Studies Using the TDT*

The inventor's calculations and observations about the increased power of the TDT in more common, less optimal situations led him to the conclusion that the power of linkage studies using the TDT is greatly increased under some conditions. Under some conditions, the power of the TDT in a linkage study using bi-allelic markers is greatly increased when each of one or more of the bi-allelic markers used in the study fulfill two criteria: (1) the allele frequencies of each of the one or more of the bi-allelic markers are similar (but not necessarily the same, or even approximately the same) as the allele frequencies of an unknown bi-allelic gene causing a disease under study; and (2) each of the one or more bi-allelic markers is in some degree of linkage disequilibrium with the gene. Thus for a typical linkage study using bi-allelic markers and the TDT, *to increase the likelihood of conditions occurring that increase the power of the TDT In the linkage study, the bi-allelic markers used in the study are chosen so that the least common allele frequencies of the markers vary systematically over a range or subrange of least common allele frequency.* This major conclusion of the inventor's research is quoted directly from his unpublished manuscript that was included with previously filed U.S. Provisional Patent Applications:"This example is typical and highlights perhaps the most important finding of this paper; namely the importance of using bi-allelic markers with heterozygosity similar to that of a bi-allelic disease gene. Indeed, since a majority of susceptibility loci may be bi-allelic, the judicious use of bi-allelic markers of both high, medium and low heterozygosity may be crucial in order to detect and replicate linkages to loci conferring modest disease risk:" (page 25) (In this context the phrase "bi-allelic markers with heterozygosity similar to that of a bi-allelic disease gene" is essentially equivalent to "bi-allelic markers with individual allele frequencies similar to those of a bi-allelic disease gene" and "bi-allelic markers of both high, medium and low heterozygosity" is essentially equivalent to the phrase "bi-allelic markers whose least common individual allele frequencies are high, medium and low".)

## Systematically Varying Both Marker Chromosomal Location and Marker Allele Frequency of Markers in Linkage Studies

The inventor's calculations and observations have demonstrated the increased power of the TDT in more common, less optimal situations when a bi-allelic marker and bi-allelic gene have (1) similar but not identical allele frequencies and (2) the marker and gene are in some degree of linkage disequilibrium. Thus, for a typical linkage study using bi- allelic markers and the TDT, *to increase the likelihood of both criteria (1) and (2) occurring for one or more markers, so as to increase the power of the TDT in the linkage study, the bi-alielic markers used in the study are chosen so that the least common allele frequencies of the markers vary systematically over a range or subrange of least common allele frequency AND the chromosomal location of the markers vary systematically over one or more chromosomes or chromosomal regions. And the bi-allelic markers are chosen so that the markers' chromosomal locations and least common allele frequencies vary systematically in an essentially independent manner.*

## Two-dimensional Linkage Study Techniques

As has been stated, conventional linkage study scanning techniques use markers that are distributed approximately evenly in the dimension of chromosomal location. These conventional, one dimensional, scanning techniques focus primarily on the chromosomal location of markers used in a scan and give little attention to the dimension of allele frequency. [1,2,3]

One of the main implications of the inventor's work is to use a set of bi-allelic markers for a typical linkage study using the TDT (or other association-based linkage test) wherein the chromosomal locations and least common allele frequencies of the markers in the set systematically vary in an essentially independent manner over the dimensions of chromosomal location and least common allele frequency respectively. This is equivalent to using a set of bi-allelic markers for a linkage study scan wherein the set of markers systematically scan or "cover" a two-dimensional region having dimensions of chromosomal location and least common allele frequency. (Such a two-dimensional region can be thought of as an area in an x-y plot or a group of squares on a chessboard.)

In addition, the inventor's calculations and observations indicate that bi-allelic markers having least common allele frequencies less than 0.3, 0.2 or even less than 0.1 have an important place in linkage studies using association based linkage tests. This is markedly different than Kruglyak's information content evaluation of bi-allelic markers for use in linkage studies, in which bi-allelic markers with least common allele frequencies less than 0.3 or 0.2 are viewed unfavorably.[4]

*In addition, the two-dimensional linkage study techniques do not necessarily favor using markers in a scan that are about evenly spaced along a chromosome* as *in the conventional techniques.* ***This is because conventional techniques suffer from a kind of one dimensional view or lack of depth perception.*** **In the conventional techniques, a marker can look very close to a gene's location in terms of chromosomal location, but the marker can be very far from the gene's location in the new two dimensional view used by versions of the invention.**

**It is as if the conventional 1D techniques look at a chessboard from on edge. Markers and a gene which are on different squares of the board, but in the same column of squares, look very close to each other when the board is looked at from on edge. But when the board is looked at from the top in 2D, two dimensions, markers which looked very close to each other and the gene before (when looking from on edge) can be seen to be very far from the gene.**

Further Implications of the Two-dimensional Linkage Study Perspective

These two-dimensional techniques work when multiple genes cause a genetic characteristic and are effective in searching for these genes. A two-dimensional bi-allelic marker "covering" or scanning approach also increases the power of linkage studies using other association based linkage tests such as the AFBACmethod, the haplotype relative risk (HRR) method [12], and comparison of marker allele frequencies in disease cases and unrelated controls[13].

**Other Background Art**

Lindlöf, et. al, Linked polymorphic DNA markers in the prediction of X-linked muscular dystrophy. Annals of Human Genetics 1987 Oct;51 (Pt 4):317-28. In this reference ten polymorphic DNA markers, including gene specific markers of loci DXS164 and DXS206, were tested for allele frequencies, degree of heterozygosity and linkage in 34 Finnish families with X-linked muscular dystrophy.

Ramsay, et. al., Haplotype analysis to determine the position of a mutation among closely linked DNA markers. Human Molecular Genetics 1993 Jul;2(7):1007-14. Using cystic fibrosis in European populations as a model system, methods for determining the position of a mutation are discussed. These methods predict the location of the major CF mutation accurately from a real set of more than 600 European CF chromosomes.

Devlin B, & Risch N, A comparison of linkage disequilibrium measures for fine-scale mapping. Genomics. 1995 Sep 20;29(2):311-22. Simple disequilibrium mapping is an approach to localizing a disease locus by plotting disequilibrium values against marker locations. The simple mapping properties of five measures of disequilibrium are investigated. Collins & Morton, Mapping a disease locus by allelic association. Proceedings of the National Academy of Sciences of the U S A. 1998 Feb 17;95(4):1741-5.

A composite likelihood estimate of location for a disease gene against a high-resolution marker map by using allele frequencies at linked loci is described. The method is illustrated by analysis of published cystic fibrosis haplotypes.

**Inventor(s): COHEN & BLUMENFELD Method for generating a high density linkage disequilibrium-based map of the human genome.** Publication number: EP0892068 (A1) Publication date: 1999-01-20. Methods are described for generating a high density linkage disequilibrium map of the human genome, markers obtained by the methods, and probes, primers and arrays capable of detecting the markers as well as diagnostic assays using the probes and genes identified by the methods.

**Inventor(s): SCHORK, ET.AL. Methods, software and apparati for identifying genomic regions harboring a gene associated with a detectable trait.** Publication number: WO0028080 (A2), Publication date: 2000-05-18. The invention relates to methods, software, and apparati for determining whether a genomic region harbors a gene associated with a detectable trait. One embodiment of the invention relates to a method of using biallelic markers.

Chee, et. al. Accessing genetic information with high-density DNA arrays. Science. 1996 Oct 25;274(5287): 610-4, and Wang, et. al., Large-scale identification, mapping, and genotyping of single-nucleotide polymorphisms in the human genome. Science. 1998 May 15;280(5366):1077-82. These two references (Chee and Wang) describe arrays of large numbers of oligonucleotide probes that are useful for obtaining various kinds of genetic information.

Patents That May Be Helpful in Starting A Search Of The Background

Some patents that are in the same general areas as versions of the invention are cited here: US Patent Number 5,667,976 Solid supports for nucleic acid hybridization assays. Published international Application WO 98/20165 Biallelic Markers. Published International Application WO 98/07887 Methods for treating bipolar mood disorder associated with markers on chromosome 18 p. US Patent Number 5,552,270 Methods of DNA sequencing by hybridization based on optimizing concentration of matrix-bound oligonucleotide and device for carrying out same. No patent in this paragraph is admitted to being prior art with respect to the present invention by it's mention in this background.

Yershov, et. al., DNA analysis and diagnostics on oligonucleotide microchips. Proceedings of the National Academy of Sciences of the U S A. 1996 May 14;93(10):4913-8.

Guo, et. al., Direct fluorescence analysis of genetic polymorphisms by hybridization with oligonucleotide arrays on glass supports. Nucleic Acids Res. 1994 Dec 11;22(24):5456-65.

Drobyshev, et. al., Sequence analysis by hybridization with oligonucleotide microchip: identification of beta-thalassemia mutations. Gene. 1997 Mar 25;188(1):45-52.

These three references (Yershov, Guo, and Drobyshev) also describe arrays of large numbers of oligonucleotide probes that are useful for obtaining various kinds of genetic information.

No patent or reference, including any "Other Background Art" is admitted to being prior art with respect to the present invention by its mention in this Background.

The following references are cited in the above paragraphs:

1 Reed, et. al. : Chromosome-specific microsatellite sets for fluorescence-based, semi-automated genome mapping. Nature Genetics, July 1994; vol. 7: pp.390-395.

2 Levinson, et. al. Genome Scan of Schizophrenia. Am J Psychiatry, June 1998; vol. 155: pp. 741-750.

3 Kruglyak, et al.: Linkage Thresholds for Two-stage Genome Scans. Am J Hum Genet, 1998. vol. 62: pp. 994-996.

4 Kruglyak: The use of a genetic map of biallelic markers in linkage studies. Nature Genetics. September 1997, vol. 17, pp.21-24.

5 Kruglyak, et al.: Complete Multipoint Sib-Pair Analysis of Qualitative and Quantitative Traits. Am J Hum Genet, 1995. vol. 57: pp. 439-454.

6 Kruglyak, et al.: Parametric and Nonparametric Linkage Analysis. Am J Hum Genet, 1996. vol. 58: pp. 1347-1363.

7 Spielman, R.S., McGinnis, R.E., Ewens, W.J.: Transmission Test for Linkage Disequilibrium: The Insulin Gene Region and Insulin-dependent Diabetes Mellitus(IDDM). Am J Hum Genet, 1993. vol. 52: pp. 506-516.

8 Risch, N. and Merikangas, K.: The Future of Genetic Studies of Complex Human Diseases. Science, 13 September 1996. vol.273, pp. 1516-1517.

9 Muller-Myshok, B. and Abel, L.: Technical Comments: The Future of Complex Diseases. Science. 28 February 1997. vol. 275, pp. 1328-1329.

10 Risch, N. and Merikangas, K.: Technical Comments: The Future of Complex Diseases. Science. 28 February 1997. vol. 275, p. 1330.

11 MC Ginnis R.E.: Hidden Linkage: Comparison of the affected sib pair (ASP) test and transmission disequilibrium test (TDT). Annals of Human Genetics. 1998. vol. 62. pp. 159-179.

12 Falk CT and Rubenstein P: Haplotype relative risks: an easy reliable way to construct a proper control sample for risk calculations. Annals of Human Genetics. 1987. vol. 51, pp. 227-233.

13 Bell GI, Horita S and Karam JH: A polymorphic locus near the human insulin gene is associated with insulin-dependent diabetes mellitus. Diabetes. 1984. vol. 33, pp. 176-183.

## Two-Dimensional Linkage Study Techniques

## Brief Description of Some Concepts Used By Versions of the Invention

Versions of the present invention make use of the novel concept of systematically covering a region on a two-dimensional map similar to an x-y graph with bi-allelic markers. The x axis on this map is the chromosomal location dimension and the y axis of the map is the least common allele frequency dimension. This two-dimensional map is called a CL-F map in this application. (CL stands for chromosomal location and F stands for least common allele frequency.) Each point on a CL-F map has two coordinates: a chromosomal location coordinate and a frequency coordinate. A point on a CL-F map is called a CL-F point.

Any one bi-allelic polymorphism (marker or gene) is viewed as being located at a particular CL-F point on a CL-F map. The chromosomal location of the polymorphism is the chromosomal location coordinate of the point. And the least common allele frequency of the polymorphism is the frequency coordinate of the point. The chromosomal location coordinate of a CL-F point is given in units of centiMorgans or base pairs or an equivalent thereof and the least common allele frequency coordinate of a CL-F point is given in units between 0 and 0.5 inclusive, such as 0.2.

Distances between any two CL-F points on a CL-F map are given in terms of two numbers: chromosomal location distance and frequency distance. The first number is the distance in the horizontal, chromosomal location direction. This first number is the chromosomal location distance. The second number is the distance in the vertical, frequency direction. This second number is the frequency distance. For example, the CL-F distance $\delta$ is given by two numbers $\delta_{CL}$ (chromosomal location distance) and $\delta_F$ (frequency distance). This is represented as $\delta = [\delta_{CL,} \, \delta_F]$.

The "clustering" of bi-allelic markers near a particular CL-F point is discussed in terms of the number of markers within a particular CL-F distance of the point. For example, if each of N bi-allelic markers is separated from the point by a CL-F distance of less than or equal to $\delta$, then the point is said to be N covered by the markers to within the distance $\delta$. (N being an integer number.)

A region on a CL-F map is called a CL-F region. A CL-F region is a collection of one or more CL-F points. Some systematic methods of covering a CL-F region with bi-allelic markers are discussed in terms of the number of markers that are near each point in the region. For example, if each CL-F point in a CL-F region is N covered to within a CL-F distance $\delta$ by a subset of a set (or group) of bi-allelic markers, then the region is said to be N covered by the set (or group) of bi-allelic markers to within the distance $\delta$.

A set (or group) of bi-allelic markers that cover a CL-F region or a CL-F point is referred to as a set (or group) of bi-allelic covering markers in this application.

The inventor discovered that when a bi-allelic marker and a bi-allelic gene are located close together on a CL-F map, then the power of association based linkage tests to detect linkage disequilibrium between the marker and a trait-causing gene (when present) increases greatly. Systematically covering a CL-F region that is the location of an unknown trait-causing bi-allelic gene with bi-allelic covering markers, therefore greatly increases the power of association based linkage tests to detect linkage disequilibrium (when present) between one or more of the covering markers and the gene.

A CL-F matrix is a matrix of rectangular cells of the same length and the same width on a CL-F map. Stipulations that a certain number of covering markers are placed in each cell of the matrix is a method of illustrating particular types systematic covering of a CL-F region with covering markers.

The evidence for linkage obtained from two-dimensional linkage studies is essentially two-dimensional in nature and it is possible to use this two-dimensional information by essentially graphing quantitative evidence for linkage as a function of position in the x-y plane. For example, if quantitative evidence for linkage is represented in the z dimension of a typical three-dimensional x-y-z plot, wherein the x and y dimensions are chromosomal location and least common allele frequency respectively, then it is possible to conceptualize evidence for linkage as occurring in a "hump" or "humps" in the z dimension. And it is possible to analyze the data to find the CL-F location (in the x-y plane) of the peak(s) of this "hump(s)", thus helping to localize a trait causing gene to the CL-F locale of the peak(s) of the "hump(s)".

Versions of the invention also make use of multi-allelic genes and/or markers. It is always possible to combine the alleles of a multi-allelic polymorphism (marker or gene) so that the polymorphism acts mathematically like it is a bi-allelic polymorphism. In effect, it is always possible to mathematically transform a multi-allelic marker or gene to act bi-allelic. Similarly, two or more markers can always be mathematically combined to form a mathematical marker that acts like a single bi-allelic marker. And two or more genes can always be mathematically combined to form a mathematical gene that acts like a single bi-allelic gene. In this application a mathematical bi-allelic marker formed mathematically from one or more markers is called a bi-allelic marker equivalent or BME; and a mathematical bi-allelic gene formed mathematically from one or more genes is called a bi-allelic gene equivalent or BGE.

The term true marker or gene is used to distinguish a marker or gene in the ordinary sense from a bi-allelic marker equivalent (BME) or bi-allelic gene equivalent (BGE). The term true allele is used to distinguish an allele in the ordinary sense from a mathematical allele of a BME or BGE. A mathematical allele of a BME or BGE is referred to as an allele equivalent. An allele equivalent is a combination of one or more true alleles or one or more haplotypes.

Versions of the invention make use of genes and/or markers, which are not exactly bi-allelic. These genes or markers are approximately bi-allelic. A gene or marker that is approximately bi-allelic almost always occurs in one of two allele forms, however, very rarely it occurs in a different allele form. Various versions of the invention are for genotyping individuals at markers which systematically cover CL-F regions or for obtaining sample allele frequency data (such as from pooled DNA) for a sample of individuals for markers which systematically cover CL-F regions. Various versions of the invention are for oligonucleotides used for genotyping individuals at markers which systematically cover CL-F regions or are for obtaining sample allele frequency data (such as from pooled DNA) for a sample of individuals for markers which systematically cover CL-F regions.

## Definitions

For the purposes of the description and claims the terms used herein will have their generally accepted definition unless otherwise specified.

The term **creature** means any organism that is living or was alive at one time. This includes both plants and animals.

The term **species** is used in it's broadest sense and includes but is not limited to : 1) biological (genetic) species,2) paleospecies (successional species), 3) taxonomic (morphological ; phenetic) species including species hybrids such as mules, 4) microspecies (agamospecies) 5) biosystematic species( coenospecies,ecosystem species)

A **genetic characteristic** is an observable or inferable inherited genetic characteristic or inherited genetic trait including a biochemical or biophysical genetic trait, for example an inherited disease is a genetic characteristic, a predisposition to an inherited disease is a genetic characteristic. A phenotypic characteristic, phenotypic property or character is a genetic characteristic.

In this application, **the term gene** means a polymorphism that takes on one or more allele forms and which causes or determines an inherited genetic characteristic or genetic trait. **The term gene** does not mean an entire gene structure with a promoter region, a terminator region, introns, and other parts of an entire gene structure. In this application the term gene means a polymorphism that determines or causes an inherited genetic characteristic and that is part of an entire gene structure in some cases. Each **genetic characteristic** of a creature is **determined** by one or more of the creature's **genes,** wherein the term gene is defined as above.

A **segment** is a segment of a chromosome.

A **subrange** is a subrange of the least common allele frequency range 0 to 0.5 inclusive.

The **width** of a subrange is the difference between the upper and lower limits of the subrange. For example, the width of the subrange 0.1 to 0.4 is 0.4 - 0.1 = 0.3.

A **chromosomal location-least common allele frequency map** is a two-dimensional plot (similar to an x-y graph) wherein the vertical axis(y axis) represents least common allele frequency and the horizontal axis(x axis) represents chromosomal location. A chromosomal location-least common allele frequency map is referred to as a **CL-F map.**

**Points on a CL-F map are referred to as CL-F points.** Points on a CL-F map have a chromosomal location coordinate and a least common allele frequency coordinate. CL-F points represent possible chromosomal location and least common allele frequency values for individual bi-allelic markers and genes. Any particular point on a CL-F map is directly opposite a value on the map's least common allele frequency axis(y axis) and is directly opposite a value on the map's chromosomal location axis(x axis). These two values are the two coordinates of the particular point: (1) the chromosomal location coordinate and (2) the least common allele frequency coordinate. A marker or gene located at a particular point on a CL-F map is physically located at the chromosomal location given by the chromosomal location coordinate of the point and the marker or gene's least common allele frequency is the least common allele frequency coordinate of the point. These two coordinates are designated by the term ( x, y ) wherein x is the value of the chromosomal location coordinate and y is the value of the least common allele frequency coordinate.

**A particular CL-F map may be large** or **small.** For example it is possible for the chromosomal location coordinates of CL-F points on a particular CL-F map to range over an entire chromosome ( for example human chromosome number 6). Alternatively it is possible for the chromosomal location coordinates of CL-F points on a particular CL-F map to range over more than one chromosome, for example all the human chromosomes, human chromosomes numbers1 through 22 and X and Y. Similarly it is possible for the chromosomal location coordinates of CL-F points on a particular CL-F map to range over all the chromosomes of a species under study. Alternatively, it is possible for the chromosomal location coordinates of CL-F points on a particular CL-F map to range over a very small segment of chromosome, for example a segment of length 100,000 bp or less. Similarly it is possible for the least common allele frequency coordinates of CL-F points on a particular CL-F map to range over the entire least common allele frequency range 0 to 0.5. Alternatively it is possible for the least common allele frequency coordinates of CL-F points on a particular CL-F map to range over a subrange or subranges of the range 0 to 0.5, for example the subrange 0.1 to 0.2.

If **a bi-allelic polymorphism (marker or gene) is said to be located at a particular CL-F point** then the polymorphism's chromosomal location is the chromosomal location coordinate of the point and the polymorphism's least common allele frequency is the least common allele frequency coordinate of the point.

**The chromosomal location distance between two CL-F points on a CL-F map** is the absolute difference between the two chromosomal location coordinates of the two points.

**The frequency distance between two CL-F points** on a CL-F map is the absolute difference between the two least common allele frequency coordinates of the two points.

**The CL-F distance between two CL-F points** is given in terms of two parts or two components : (1) chromosomal location distance and (2) frequency distance. This is denoted as $[D_{CL}, D_F]$, wherein $D_{CL}$ is the chromosomal location distance between the two points and $D_F$ is the frequency distance between the two points. For example [500 bp, 0.3 ] is an example of a CL-F distance.

If **a first CL-F distance is less than or equal to a second CL-F distance** then the chromosomal location distance component of the first CL-F distance is less than or equal to the chromosomal location distance component of the second CL-F distance AND the frequency distance component of the first CL-F distance is less than or equal to the frequency distance component of the second CL-F distance. For example if a first CL-F distance is $[x_1, y_1]$ and a second CL-F distance is $[x_2, y_2]$. And if the first CL-F distance is said to be less than or equal to the second CL-F distance, then x, is less than or equal to $x_2$ AND $y_1$ is less than or equal to $y_2$.

**The term "bi-allelic covering marker(s)" or "covering marker(s)"** is used to distinguish a particular bi-allelic marker or particular bi-allelic markers from other markers. The term is being used simply to avoid ambiguity. In general the term covering marker(s) can be thought of as a marker or markers which have been chosen to cover or serve to cover a CL-F point or a CL-F region.

If **a CL-F point is said to be N covered to within a CL-F distance** $\delta$ **by one or more bi-allelic covering markers** then the CL-F distance between each of N or more of the covering markers and the point is less than or equal to $\delta$. Wherein N is an integer number greater than or equal to 1.

If **a CL-F point is said to be N covered to within a CL-F distance of about (or approximately)** $\delta$ **by one or more bi-allelic covering markers** then the CL-F distance between each of N or more of the covering markers and the point is less than or equal to about (or approximately) $\delta$. Wherein N is an integer number greater than or equal to 1.

**A CL-F region** is a group of CL-F points. A CL-F region is a region that is or can be represented on a CL-F map. A particular CL-F region may be large or small. For example the chromosomal location coordinates of CL-F points in a particular CL-F region can range over an entire chromosome ( for example human chromosome number 6). Alternatively the chromosomal location coordinates of CL-F points in a particular CL-F region can range over more than one chromosome, for example all the human chromosomes, human chromosomes numbers 1 through 22 and X and Y. Similarly the chromosomal location coordinates of CL-F points in a particular CL-F region can range over all the chromosomes of a species under study. Alternatively, the chromosomal location coordinates of CL-F points in a particular CL-F region can range over only a small segment of chromosome, for example a segment of length 100,000 bp or less. Similarly the least common allele frequency coordinates of CL-F points in a particular CL-F region can range over the entire least common allele frequency range 0 to 0.5. Alternatively the least common allele frequency coordinates of CL-F points in a particular CL-F region can range over only a very small subrange, for example the subrange 0.1 to 0.2 or less.

**The length of a CL-F region** is the largest chromosomal location distance between any two CL-F points in the region.

**The width of a CL-F region** is the largest frequency distance between any two CL-F points in the region.

**A CL-F region that is path connected** is contiguous and it is possible to draw a continuous path between any two points, wherein each point in the path is also in the region.

If **a CL-F region is said to be systematically covered by two or more bi-allelic covering markers** then each point in the region is within a small CL-F distance of one or more of the covering markers, wherein the magnitude of the small CL-F distance is such that there is increased power of an association based linkage test to detect evidence for linkage between one or more covering markers and a gene that is located at a point in the CL-F region, when linkage disequilibrium is present between the gene and one or more of the covering markers.

If **a CL-F region is said to be N covered to within a CL-F distance** $\delta$ **by one or more covering markers** then each point in the region is N covered to within the CL-F distance $\delta$ by the one or more covering markers. Wherein N is an integer greater than or equal to one.

If **a CL-F region is said to be N covered to within a CL-F distance of about (or approximately)** $\delta$ **by one or more covering markers** then each point in the region is N covered to within the CL-F distance of about (or approximately) $\delta$ by the one or more covering markers. Wherein N is an integer greater than or equal to one.

**The CL-F distance** $\delta$ **is known as the covering distance** if a CL-F point or CL-F region is N covered to within a CL-F distance $\delta$.

**A CL-F covering distance** $\delta$ **has two components:** (1) a chromosomal location distance usually denoted by $\delta_{CL}$ and (2) a least common allele frequency distance (abbreviated as frequency distance) usually denoted by $\delta_F$, i.e. $\delta = [ \delta_{CL}. \delta_F ]$.

The **length of a group of covering markers** is determined as follows. The absolute chromosomal location distance between each pair of markers in the group is determined. The greatest absolute chromosomal location distance between each pair of markers in the group is the length of the group of covering markers.

**A group of covering markers located on one chromosome can be ordered as a sequence of markers** starting with the marker closest to one end of the chromosome and going toward the other end of the chromosome. This is denoted for example as $m_1. m_2, m_3, ...., m_{N-2}, m_{N-1}, m_N$, wherein N is the number of markers in the group. (The chromosomal location distance between $m_1$ and $m_N$ is greater than the chromosomal location distance between any other pair of markers in the group and this distance is the length of the group of markers.) **The chromosomal location distance between two successive markers in the group**, i.e. between $m_R$ and $m_{R+1}$, is a **chromosomal intermarker distance.** (There are N-1 chromosomal intermarker distances for a group of N covering markers.) **The average chro-**

**mosomal intermarker distance** for a group is calculated by dividing the length of the group by (N-1), wherein N is the number of covering markers in the group.

**The width of a CL-F region** is the largest frequency distance between any two CL-F points in the region.

**The length of a CL-F region** is the largest chromosomal location distance between any two CL-F points in the region.

A **segment-subrange pair** is the pair formed by pairing a segment of a chromosome and a subrange of the least common allele frequency range 0 to 0.5.

The **term segment-subrange** is used as a short version of the term segment-subrange pair. (A segment-subrange is a rectangular region on a CL-F map or a rectangular CL-F region, see below.) If **one or more bi-allelic markers are said to be within(or in) a segment-subrange** then each of the markers is located on (or in) the chromosomal segment of the segment-subrange(pair) and each of the markers' least common allele frequencies is in the subrange of the segment-subrange(pair). (And each of the markers is located within the rectangular region defined by the segment-subrange on a CL-F map.)

Alternatively, if **a segment-subrange is said to contain one or more markers or to contain the location of one or more markers** then each of the markers is located on (or in) the chromosomal segment of the segment-subrange and each of the markers' least common allele frequencies is in the subrange of the segment-subrange. (And each of the markers is located within or is within the rectangular region on a CL-F map defined by the segment-subrange.)

If **one or more CL-F points are said to be within(or in) a segment**-subrange then each of the points is located within the rectangular region defined by the segment-subrange on a CL-F map or on the segment-subrange's borders.

**The length of a segment-subrange** is the length of the segment of the segment-subrange.

**The width of a segment-subrange** is the width of the subrange of the segment-subrange.

**The area of a segment-subrange** is the segment subrange's length multiplied by the segment subrange's width.

If **a CL-F region is said to comprise a segment-subrange**, then each point in the segment-subrange is in (or included in) the CL-F region.

If **a CL-F region is said to comprise an area of greater than or equal to X multiplied by Y**, then the CL-F region comprises one or more nonoverlapping segment-subranges, and the sum of the areas of the segment-subranges is greater than or equal to X multiplied by Y.

A **CL-F matrix** is a collection of segment-subranges, wherein each segment-subrange of the collection has the same width and the same length. Each segment-subrange in the collection (or the matrix) is a **CL-F matrix cell.** Any one CL-F matrix cell in a CL-F matrix shares two or more of the cell's borders with two or more other cells in the matrix. And all the cells in a CL-F matrix together form a single segment-subrange. A CL-F matrix is characterized by the length and the width of the cells in the denoted by length x width, or $L_{MC}xW_{MC}$, wherein $L_{MC}$ is the length of each cell in the matrix and $W_{MC}$ is the width of each cell in the matrix. A CL-F matrix is also characterized by the number of rows of cells, $R_M$, in the matrix. And a CL-F matrix is characterized by the number of columns of cells, $C_M$, in the matrix. There are two or more cells in a CL-F matrix. A CL-F matrix is also characterized by the point of origin of the matrix, denoted by $(cl_O, f_O)$. The point of origin of a CL-F matrix is at any chromosomal location and $cl_O$ takes on any reasonable value in an entire species genome. The point of origin of a CL-F matrix is at any one value in the least common allele frequency range 0 to 0.5. (A CL-F matrix is similar to the squares of a chessboard or to equal rectangular floor tiles that are all oriented in the same direction and cover a rectangular floor. One corner of the matrix is the matrix's point of origin.)

The **width of each cell of a particular CL-F matrix** is any value greater than zero and less than 0.5. The width of a cell is often denoted by $W_{MC}$.

Any length in chromosomal location distance units is chosen for the **length of each cell of a particular CL-F matrix.** The length of a cell is often denoted by $L_{MC}$.

The **centerpoint** of a CL-F matrix cell is in the center of the cell. The centerpoints of a CL-F matrix form a **matrix centerpoint lattice.** Each point of a matrix centerpoint lattice is separated by a CL-F distance of $[0, W_{MC}]$ or $[L_{MC}, 0]$ from two or more neighboring centerpoints.

If **one or more bi-allelic markers are in(or within) the segment-subrange that is a CL-F matrix cell,** then each of the markers is in or within the CL-F matrix cell.

If **one or more CL-F points is in (or within) a CL-F matrix,** then each of the points is in or within a cell of the matrix.

If **a CL-F region comprises a CL-F matrix,** then each point that is in the matrix is also in the region.

If **a CL-F region is a CL-F matrix,** then the region consists of the points that are in the matrix.

If two CL-F matrix cells share a common border, then the **two CL-F matrix cells are in contact.**

If two CL-F matrix cells share a common corner, then the **two CL-F matrix cells are touching**. (Two cells that are in contact are also touching.)

If **a group of CL-F points is connected to within a CL-F distance [X,Y],** then for any two points in the group,

denoted $p_1$ and $p_R$ , there is an ordered sequence of points in the group denoted $p_1$, $p_2$, $p_3$,....., $p_{R-2}$, $p_{R-1}$, $p_R$ , R being an integer greater than or equal to 2, wherein the CL-F distance between each point in the sequence and the next point in the sequence is less than or equal to [X,Y]. **The distance [X,Y] is the connecting distance.** (Put in simple terms if a group of points is connected to within [X,Y], then there is a path between each pair of points in the group, the path consisting of a series of steps, wherein each step in the path is a movement between two points in the group that are separated by a CL-F distance of less than or equal to [X,Y]. A simple group of points connected to within a CL-F distance of [X,Y] is a group of three points, wherein each point in the group is within a CL-F distance of less than or equal to [X, Y] of another point in the group. The concept of connectivity introduced here is similar to the basic concept of connectivity in mathematical graph theory.)

If **a group of N markers is connected to within a CL-F distance [X,Y],** wherein N is an integer, then each of the markers is located at one point of group of N points, the group of N points being connected to within a CL-F distance [X,Y].

If **two bi-allelic markers are said to be in extreme positive disequilibrium** then d is approximately equal to $d_{max}$ for the two markers, which for the purposes of this definition are designated marker M with least common allele A and marker m with least common allele B. Wherein according to standard usage, the disequilibrium coefficient (d) is defined by the equation d=f(AB) - f(A)f(B) where f(A) and f(B) are defined as the population frequencies of alleles A and B, respectively, and f(AB) is the population frequency of the AB haplotype. And $d_{max}$ is defined as the maximum possible positive value of d assuming the allele frequencies of A and B are f(A) and f(B), and thus $d_{max}$= q-f(A)f(B) where q is the lesser of f(A) and f(B). (In this application d is used to represent the disequilibrium coefficient; the symbol δ is often used in scientific papers to represent the disequilibrium coefficient.)

If **a pair of markers is said to be in extreme positive disequilibrium**, then the two markers of the pair are in extreme positive disequilibrium.

If **a pair of bi-allelic markers is said to be redundant within distance D** then the two markers of the pair are in extreme positive disequilibrium and the two markers are located on the same chromosome and the two markers are located within a CL-F distance D of each other on a CL-F map, wherein D is a specified distance and D has two components, a chromosomal location distance component $D_{CL}$ and a frequency distance component, $D_F$; D = [$D_{CL}$, $D_F$ ].

**An allele equivalent (AE)** is a group of one or more "haplotype values" of one or more polymorphisms of the same type, either markers or genes. ( For the purposes of this application a haplotype value of one polymorphism is equivalent to an allele value at the one polymorphism.) The group of haplotype values is then analyzed as if the group is a single allele at a bi-allelic polymorphism; the group of haplotype values acts as a single allele at a bi-allelic polymorphism; the collection of the one or more polymorphisms upon which the haplotype values are based acts as a bi-allelic polymorphism; the collection of one or more polymorphisms forms a bi-allelic **polymorphism equivalent (PE)** that acts as a bi-allelic polymorphism; **the polymorphism equivalent has(or possesses) the allele equivalent.** The allele equivalent belongs to the polymorphism equivalent. In this application, **each polymorphism equivalent is a bi-allelic marker equivalent(BME) or a bi-allelic gene equivalent(BGE).**

**A bi-allelic marker equivalent (BME)** is one or more markers and a grouping of the haplotype values of the one or more markers into two groups (e.g. group I and group II)(For the purposes of this application a "haplotype value" of one marker is equivalent to an allele at the one marker). The one or more markers and the two groups of haplotype values of the one or more markers are then analyzed as if the one or more markers are a single bi-allelic marker with alleles I and II. Each group of the groups I and II is an allele equivalent. For example, a multi-allelic microsatellite marker has it's multiple alleles grouped into two groups and the microsatellite marker and these two groups of alleles then act equivalent to a bi-allelic marker and are analyzed as if the microsatellite marker with the two groups is bi-allelic (for an example of this see McGinnis, Ewens & Spielman, Genetic Epidemiology 1995 ; 12(6) : 637-40.

Also for example, two or more multi-allelic markers have their haplotypes separated into two groups of haplotypes and the multi-allelic markers with their two groups of haplotypes are analyzed as if they were a single bi-allelic marker.

For example bi-allelic marker A has alleles a and a* and bi-allelic marker B has alleles b and b*. Then the four haplotypes ab, ab*, a*b* and a*b are grouped into two groups, for example group I: ab and a*b* and group II : ab* and a*b. Then a BME formed by markers A and B takes on values of group I (or I) for haplotypes ab or a*b* or group II (or II) for the haplotypes ab* or a*b ; and the two markers and the two group values( I and II) are analyzed as though they form a single bi-allelic marker(the BME). The same type of reasoning and procedure is extended to 3 or more bi-allelic markers, 3 or more bi-allelic marker equivalents or 2 or more multi-allelic markers.

(Logically, of course, the genotype at a BME for an individual is determined by knowing the two haplotype values at the one or more markers that form the BME for each of the individual's two homologous chromosomes that carry the one or more markers. The genotype is then determined by classifying each haplotype as belonging to group I or group II or the equivalent thereof. The three possible genotype values at the BME are I / I, I / II, and II / II or the equivalent thereof.)

Similarly, **a bi-allelic gene equivalent (BGE)** is one or more genes and a grouping of all the haplotype values of the one or more genes into two groups (e.g. group I and group II).

For the purposes of the description and claims, **the chromosomal location of a polymorphism equivalent** is at any point on the smallest chromosomal segment that contains the one or more polymorphisms that form the polymorphism equivalent(PE).

**The allele frequency of an allele equivalent (AE)** is determined as follows. An allele equivalent (AE) is a group of haplotype values of one or more polymorphisms. The frequency of the allele equivalent is the sum of the frequencies of the haplotype values in the group that makes up the allele equivalent. For the purposes of the application, description, claims and definitions the term **true allele** is used to distinguish an allele according to standard usage (i.e. at a single polymorphism) from an allele equivalent (AE).

**The least common allele frequency of a bi-allelic polymorphism equivalent (BPE)** is determined as follows. Each of the two groups( I and II) of the haplotype values of the one or more polymorphisms which form the BPE is assigned a frequency. The frequency of I is the sum of the frequencies of the haplotype values in group I. And the frequency of II is the sum of the frequencies of the haplotype values in group II. The least of the frequency of I and the frequency of II is the least common allele frequency of the BPE. If the frequency of I and the frequency of II are equal, then the least common allele frequency of the BPE is the frequency of I or the frequency of II.

For the purposes of the description and claims, **the chromosomal location of a bi-alielic marker equivalent (BME)** is at any point on the smallest chromosomal segment which contains the one or more markers which form the BME.

**The chromosomal location distance from a BME to a CL-F point** on a CL-F map is the shortest chromosomal location distance from the CL-F point to any one of the one or more markers which form the BME.

**The least common allele frequency of a bi-allelic marker equivalent (BME)** is determined as follows. Each of the two groups( I and II) of the haplotype values of the one or more markers which form the BME is assigned a frequency. The frequency of I is the sum of the frequencies of the haplotype values in group I. And the frequency of II is the sum of the frequencies of the haplotype values in group II. The least of the frequency of I and the frequency of II is the least common allele frequency of the BME. If the frequency of I and the frequency of II are equal, then the least common allele frequency of the BME is the frequency of I or the frequency of II.

**The frequency distance from a BME to a CL-F point** on a CL-F map is the absolute difference between the least common allele frequency of the BME and the least common allele frequency coordinate of the CL-F point.

( If a CL-F point on a CL-F map is covered by one or more BMEs to within a distance $\delta$, wherein $\delta = [\delta_{CL}, \delta_F]$, then the CL-F distance from each of the one or more BMEs to the CL-F point is less than or equal to $\delta$. And the chromosomal location distance from one of the markers which form each BME to the CL-F point is less than or equal to $\delta_{CL}$. And the frequency distance from each of the one or more BMEs to the CL-F point is less than or equal to $\delta_F$.)

**A bi-allelic marker equivalent is in(or within) each CL-F matrix cell that contains the chromosomal location of the bi-allelic marker equivalent (BME).** (Since the chromosomal location of a bi-allelic marker equivalent (BME) is at any point on the smallest chromosomal segment which contains the one or more markers which form the BME, in some cases, a bi-allelic marker equivalent is in more than one CL-F matrix cell.)

For the purposes of the application, the term **true bi-allelic marker** is used to distinguish a bi-allelic marker with two alleles according to usual usage (i.e. at a single polymorphism) from a bi-allelic marker equivalent(BME). A true bi-allelic marker is not a bi-allelic marker equivalent (BME). The term **true bi-allelic polymorphism** is used to distinguish a bi-allelic polymorphism with two alleles according to usual usage from a bi-allelic polymorphism equivalent(BPE).

The term **true allele** of a true bi-allelic marker means an allele of a true bi-allelic marker.

**A polymorphism(marker or gene) which is exactly bi-allelic** has exactly two alleles and the sum of the frequency of each of the two alleles is 1; for example if the two alleles are A and B, then f(A) + f(B) = 1. A polymorphism that is exactly bi-allelic is a true bi-allelic polymorphism with exactly two true alleles or a bi-alielic polymorphism equivalent (BPE) with exactly two allele equivalents.

**A polymorphism(marker or gene) which is approximately bi-allelic** has three or more alleles. And the polymorphism has a first allele and a second allele; and the sum of the frequency of the first allele and the frequency of the second allele is approximately 1. And the frequency of the first allele and the frequency of the second allele is much greater than the sum of the allele frequencies of all the alleles of the polymorphism that are not the first or the second alleles. For the versions of the invention for bi-allelic polymorphisms (bi-allelic markers and bi-allelic genes) described herein, a polymorphism which is approximately bi-allelic is analyzed as if the polymorphism has only two alleles, the first allele and the second allele. For the versions of the invention described herein, the least common allele frequency of a polymorphism which is approximately bi-allelic, is the least of the frequencies of the first and the second alleles of the polymorphism. A polymorphism which is approximately bi-allelic is a true polymorphism with true alleles (the allele frequencies of the true alleles conform to the stipulations of this definition) or is a bi-allelic polymorphism equivalent(BPE) with allele equivalents(the allele frequencies of the allele equivalents conform to the stipulations of this definition).

**SNP** stands for single nucleotide polymorphism.

**A statistical linkage test based on allelic association** is any mathematical test, mathematical computation or equivalent thereof which gives a quantitative estimate (or equivalent thereof) of evidence for linkage of a polymorphic marker and phenotypic trait (genetic characteristic) based on association between one or more of the alleles of the marker and the phenotypic trait in a sample of individuals of a population of a species. A statistical linkage test based on allelic association is any statistical test that detects or suggests linkage on the basis of allelic association. A statistical linkage test based on allelic association includes tests which suggest but do not prove linkage such as comparison of marker allele frequencies in disease cases and in unrelated controls. A statistical linkage test based on allelic association is also any test such as the TDT which may be regarded as "proving" linkage. (A statistical linkage test based on allelic association can, of course, give an estimate of the association of one or more allele equivalents of a marker equivalent and a genetic characteristic; see definition of BME above.) One aspect of a statistical linkage test based on allelic association is it's potential use to calculate the probability, or equivalent thereof, that there is genuine association of one or more of the alleles of the marker and a genetic characteristic for the population as a whole (rather than just for the sample alone). A statistical linkage test based on allelic association is an association based linkage test. (The term **population** in this application is used in a statistical sense and means a group of individuals. The term **population** in this application is not used purely in the sense the term **population** is used in the field of population genetics.)

The term **sample** means a group of individuals which is a subset of a population.

In this application, **an allele is considered to be a piece of double stranded DNA** that is singular or distinctive for the allele. The piece of double stranded DNA that is distinctive for the allele contains the particular DNA sequence that distinguishes the allele from other alleles (alternate sequences) at the polymorphic site of interest plus two double stranded "flanking" DNA sequences, one flanking DNA sequence being on one side of the polymorphic site and the other flanking DNA sequence being on the other side of the polymorphic site.

**Alternate strand of an allele:** A double stranded piece of DNA that is distinctive for an allele consists of two pieces of single stranded DNA which are exactly complementary to one another. The two pieces of single stranded DNA are referred to as the two strands of the allele. Each of the two strands of the allele is the alternate of the other strand of the allele. For the purposes of this definition, the two strands are referred to as the first strand and the second strand. The alternate strand of the first strand is the second strand. And the alternate strand of the second strand is the first strand. Each strand of an allele is exactly complementary to the strand's alternate strand.

**An oligonucleotide** is either a single or double stranded oligonucleotide. The length of an oligonucleotide ranges from a few bases or base pairs to approximately any number of bases or base pairs in the DNA sequence of any allele.

**An oligonucleotide, either single or double stranded, is complementary** to an allele if the DNA sequence of each strand of the oligonucleotide is exactly or approximately complementary to all or part of the DNA sequence of one of the DNA strands of the allele and the oligonucleotide has utility in identifying the allele by a hybridization reaction or equivalent thereof similar to as described below under oligonucleotide technology.

**An allele is identified by a hybridization reaction with an oligonucleotide that is complementary to the allele.** In this application **there are two types of oligonucleotides that are complementary to an allele.** The two types of oligonucleotides complementary to an allele are identified as type(1) or type(2).

A type (1) complementary oligonucleotide is complementary to the part of an allele's DNA sequence that actually contains the allele's polymorphic site; and the type(1) complementary oligonucleotide has utility to identify the allele by means of a hybridization reaction of the oligonucleotide to the part of the allele's DNA sequence that actually contains the allele's polymorphic site. A hybridization reaction of a type(1) oligonucleotide to the part of an allele's DNA sequence that actually contains the allele's polymorphic site is a type (1) hybridization reaction.

A type (2) complementary oligonucleotide is complementary to an allele at a DNA sequence that flanks (but does not contain) the allele's polymorphic site; and the type (2) complementary oligonucleotide has utility to identify the allele by means of a hybridization reaction wherein the oligonucleotide hybridizes to the allele at a DNA sequence that flanks (but does not contain) the allele's polymorphic site and identification of the allele is subsequently achieved by extension of the oligonucleotide (and possibly one or more other type(2)complementary oligonucleotides) across the polymorphic site with a DNA polymerase such as occurs, for example, in a standard PCR (polymerase chain reaction). A hybridization reaction of a type(2) oligonucleotide to an allele at a DNA sequence that flanks (but does not contain) the allele's polymorphic site is a type (2) hybridization reaction.

Each version of **oligonucleotide technology** is a means to test for the presence (or absence) of each of one or more true alleles of a group of true alleles in an individual's chromosomal DNA. The presence or absence of any one true allele in the group is tested for by means of a type (1) or type (2) hybridization reaction (or equivalent) with an oligonucleotide that is complementary(type(1) or type(2)) to the true allele. Put another way, the presence or absence of each true allele in the group is tested for by means of a type(1) or type(2)hybridization reaction (or equivalent) with an oligonucleotide that is complementary to each true allele in the group. There are many versions of oligonucleotide technology, some of these versions are described in more detail below. (In this application, the term "chromosomal DNA" includes chromosomal DNA obtained directly from an individual as well as DNA obtained as amplification products using PCR and chromosomal DNA obtained directly from an individual.

**A physico-chemical signal** is any physical (including chemical) signal which is detected by human senses or by apparatus. A physico-chemical signal includes, but is not limited to, (1) an electrical signal such as is generated when oligonucleotides that are attached to a silicon chip hybridize with complementary alleles, (2) a visual or optical signal such as is generated when oligonucleotides attached to a glass slide hybridize with complementary alleles, (3) a signal (such as a dye color) generated by the products of a PCR(polymerase chain reaction) such as when oligonucleotides that are used as primers for PCR reactions hybridize with complementary alleles.

**The collection of true alleles of a group of one or more bi-allelic markers** is defined as consisting of each true allele of each true marker in the group and each true allele of each haplotype that forms each allele equivalent of each marker equivalent in the group.

**If a set of oligonucleotides is said to be complementary to a group of one or more bi-allelic markers,** then each oligonucleotide in the set is type(1) or type(2) complementary to at least one of the true alleles in the collection of true alleles of the group of one or more markers; and there is an oligonucleotide in the set that is type(1) or type(2) complementary to each true allele in the collection of true alleles of the group of one or more markers.

**Sample allele frequency data for a marker and a sample** is obtained by pooling DNA specimens from individuals of the sample into one or more DNA pools. An allele frequency for each of the marker's alleles is obtained for each DNA pool. In the case of a bi-allelic marker, determining the sample allele frequency for one allele essentially determines the sample allele frequency for the other allele. (For example, in some association based linkage studies, each DNA pool contains DNA from individuals of the sample with the same or similar phenotype status.) (It is also possible to obtain sample allele frequency for a marker and a sample by calculation using genotype data at the marker for each individual in the sample.)

**Genotype data/sample allele frequency data** for a marker and a sample is (1)genotype data at the marker for each individual of the sample, or (2)a combination of genotype data at the marker for one or more individuals in the sample and sample allele frequency data for the marker for the sample, or (3)sample allele frequency data for the marker for the sample. In the case of genotype data, DNA specimens from individuals are tested individually to determine genotype. In the case of sample allele frequency data DNA specimens from individuals are pooled, or sample allele frequency is calculated using genotype data for each individual in the sample.

## Description

For the versions of the invention described herein and the claims, **a bi-allelic genetic characteristic gene or a bi-allelic gene** is a gene which is exactly bi-allelic or a gene which is approximately bi-allelic. For the versions of the invention described herein and the claims, **a bi-allelic genetic characteristic gene or a bi-allelic gene** is a gene which is a true bi-allelic gene or a bi-allelic gene equivalent (BGE). A bi-allelic gene equivalent is exactly bi-allelic or approximately bi-allelic. A true bi-allelic gene is exactly bi-allelic or approximately bi-allelic.

For the versions of the invention described herein and the claims, **a bi-allelic marker or a bi-allelic covering marker** is a marker which is exactly bi-allelic or a marker which is approximately bi-allelic. Each marker that is exactly bi-allelic is a true bi-allelic marker or a bi-allelic marker equivalent. And each marker that is approximately bi-allelic is a true bi-allelic marker or a bi-allelic marker equivalent (BME).

**Process #1,** A process for identifying one or more bi-allelic markers linked to a bi-allelic genetic characteristic gene in a species of creatures, comprising the steps of :

a)choosing two or more bi-allelic covering markers so that a CL-F region is systematically covered by the two or more covering markers;

b)choosing a statistical linkage test based on allelic association for each covering marker;

c)choosing a sample of individuals for each covering marker ;

d)obtaining genotype data/sample allele frequency data for each covering marker and the sample chosen for each covering marker, and obtaining phenotype status data for the genetic characteristic for each individual in the sample chosen for each covering marker;

e)calculating evidence for linkage between each covering marker and the gene using the statistical linkage test based on allelic association chosen for each covering marker and the genotype data/sample allele frequency data for each covering marker and using the phenotype status data for the genetic characteristic for each individual in the sample chosen for each covering marker obtained in d);

and

f)identifying those covering markers as linked to the genetic characteristic gene which show evidence for linkage based on the calculations of step e.

The following is a more detailed description of process #1.

Process #1, A process for identifying one or more bi-allelic markers linked to a bi-allelic genetic characteristic gene in a species of creatures comprising the steps of :

**a)choosing two or more bi-allelic covering markers so that a CL-F region is systematically covered by the two or more covering markers;** Any method of systematically covering the CL-F region is acceptable. In this application, the systematic covering of a CL-F region in versions of the invention is described mathematically as the covering of a CL-F region, wherein the CL-F region is N covered to within a CL-F distance δ by two or more bi-allelic covering markers. For further details regarding this step, see <u>Detailed Description of the Systematic Covering of a CL-F Region Used in Versions of the Invention</u> below. (A "set of markers" or a "group of markers," chosen for a linkage study is also called a "panel of markers" or a "marker panel.")

**b)choosing a statistical linkage test based on allelic association for each covering marker**; The statistical linkage test based on allelic association chosen for any one particular covering marker is any statistical linkage test based on allelic association as defined in the definitions section. Statistical linkage tests based on allelic association are described in the genetics and population genetics literature and are known to those of ordinary skill in the art. Some examples of a statistical linkage test based on allelic association are the TDT, Haplotype Relative Risk Method (HRR) and Allele Frequency Comparison In Disease Cases Versus Unrelated Controls .It is possible for different statistical linkage tests based on allelic association to be chosen for different covering markers. For purposes of technical convenience, the same statistical linkage test based on allelic association is preferably chosen for each covering marker.

**c)choosing a sample of individuals from the species for each covering marker**; For the process to be workable, the sample chosen for any one covering marker must be suitable for the statistical linkage test of b) above chosen for the covering marker. Knowledge of a suitable sample for the statistical linkage test chosen in b) above for the covering marker is within the understanding of a person skilled in the art. For purposes of technical convenience, the same sample of individuals is preferably chosen for each covering marker.

**d)obtaining genotype data/sample allele frequency data for each covering marker and the sample chosen for each covering marker, and obtaining phenotype status data for the genetic characteristic for each individual in the sample chosen for each covering marker;** Sample allele frequency data for any one covering marker for the sample chosen for the covering marker is obtained by pooling DNA from individuals of the sample into one or more DNA pools. It is also possible to obtain sample allele frequency data for any one covering marker by calculation using genotype data at the marker for each individual in the sample. Each DNA pool contains DNA from individuals of the sample with the same or similar phenotype status. An allele frequency for each of the marker's alleles is obtained for each pool. Genotype data/sample allele frequency data for any one covering marker is (1) genotype data at the covering marker for each individual in the sample chosen for the covering marker, or (2)a combination of genotype data at the covering marker for one or more individuals in the sample chosen for the covering marker and sample allele frequency data for the covering marker for the sample chosen for the covering marker, or (3)sample allele frequency data for the covering marker for the sample chosen for the covering marker. The genotype data/sample allele frequency data for any one covering marker must be suitable for the statistical linkage test based on allelic association chosen for the covering marker in step b). It is possible to choose different types of genotype data/sample allele frequency data for each covering marker. For purposes of technical convenience, the same type of genotype data/sample allele frequency data (1), (2), or (3) is chosen for each covering marker. Some examples of ways to practice this step is the use of technology cited under <u>Oligonucleotide Technology</u> (below) or mass spectrometry (such as MALDITOF).'

**e)calculating evidence for linkage between each covering marker and the gene using the statistical linkage test based on allelic association chosen for each covering marker and the genotype data/sample allele frequency data for each covering marker and using the phenotype status data for the genetic characteristic for each individual in the sample chosen for each covering marker obtained in d); and**

**f)identifying those covering markers as linked to the gene which show evidence for linkage based on the calculations of step e.**

The meanings of steps d, e and f are within the understanding of those of ordinary skill in the art. Fine points of using a statistical linkage test based on allelic association as a measure of evidence for linkage are known to those in the art."

Process #1 described above is equivalent to localizing a genetic characteristic gene to a particular chromosomal location (i.e. a sub-region of a particular chromosome.) This is because markers which are linked to a gene are also physically close to the gene in terms of physical (chromosomal) location. To locate a gene causing the genetic characteristic of Process #1, the gene is localized to the approximate chromosomal location of one or more covering markers which are identified as showing evidence for linkage in step f).

**Process#1A** It is also possible to use Process #1 to localize a genetic characteristic gene to an approximate CL-F location(chromosomal location-least common allele frequency location). Such a process is expressed as follows:

> **Process#1A: A process for localizing a bi-allelic genetic characteristic gene in a species of creatures to a chromosomal location-least common allele frequency (CL-F) location, comprising the steps a), b), c), d) and e) of Process #1 and further comprising the step of:**

> > **f)localizing the gene to the chromosomal location-least common allele frequency (CL-F) location of one or more markers that show evidence for linkage based on the calculations of step e).** It is the teaching of this application that the strength of evidence for linkage increases as markers that are in linkage disequilibrium with a gene become close to the gene on a CL-F map. It is possible for step f) to be done by an individual plotting data by hand and examining the data. It is also possible for software to perform step f). It is possible for this step to include using the dependence of quantitative evidence for linkage of step e) on CL-F location. For example, if quantitative evidence for linkage calculated in step e) (of process #1 or #1A) is represented in the z dimension of a typical three-dimensional x-y-z plot, wherein the x and y dimensions are chromosomal location and least common allele frequency respectively, then it is possible to conceptualize evidence for linkage as occurring in a "hump" (or "humps" )in the z dimension. And it is possible to use the evidence for linkage calculated in step e) of (process #1 or #1A) to find the CL-F location (in the x-y plane) of the peak(s) of a "hump(s)", thus helping to localize a trait causing gene to the CL-F locale of the peak(s) of the "hump(s)". For example it is possible to use computer programming techniques that detect gradients such as, for example, linear or nonlinear programming techniques in mathematical optimization theory[III] to find the peak(s) of a hump (s) in this step.

(Process #1A described above is equivalent to localizing a genetic characteristic gene to a particular chromosomal location (i.e. a sub-region of a particular chromosome.) This is because localizing a gene to a particular CL-F region also localizes the gene to a particular chromosomal region.)

## Software

A computer program that executes each step of Process#1 is an example of Process#1. A computer program that executes each step of Process#1A is an example of Process#1A. A flowsheet illustrating programs that execute Process#1 and Process#1A is entitled Drawing #1(see drawing section). It is also possible for a computer program to execute any one of(or one or more combinations of) the steps of Process#1 or Process#1A. A person of ordinary skill in the art could write such a program without undue experimentation. The level of skill at computer programming in the art is great as evidenced by numerous computer programs. Some computer programs in the art are programs such as MAPMAKER/SIBS[IV], GENEHUNTER[V], LINKAGE[VI], and FASTLINK.[VII]

Detailed Description of the Systematic Covering of a CL-F Region Used In Versions of the Invention

(see definitions section for meaning of CL-F region that is systematically covered). The CL-F region and covering markers are for a species and the one or more individuals are members of the species. The chromosomal location coordinate of each covering marker is based on information regarding the chromosomal location of each covering marker. One such source of information is chromosomal maps. Chromosomal maps are provided by such institutions as the Whitehead Institute or Marshfield Foundation for Biomedical Research. Chromosomal maps include, but are not limited to genetic maps, physical maps, and radiation hybrid maps.

The least common allele frequency coordinate of each covering marker is based on any reasonable information regarding the least common allele frequency of each covering marker. It is possible to use information from different populations for the allele frequencies of different covering markers. For example, it is possible for the least common allele frequencies of two different covering markers to be based on information from two different, but similar populations. For purposes of technical convenience, the least common allele frequency of each covering marker is based on information from the same population. One source of information on least common allele frequency is institutions which provide chromosomal maps such as the Whitehead Institute or Marshfield Foundation for Biomedical Research.

**Systematic Covering Of A CL-F Region, Wherein A CL-F Region is N Covered To Within A CL-F Distance $\delta$ By Two or more Bi-Allelic Covering Markers**

In this application, the systematic covering of a CL-F region in versions of the invention is described mathematically as the covering of a CL-F region, wherein the CL-F region is N covered to within a CL-F distance $\delta$ by two or more bi-allelic covering markers. The covering markers are chosen so that the CL-F region is N covered to within the CL-F distance $\delta$ by using information regarding the chromosomal location and least common allele frequency of each covering marker.

It is possible for the chromosomal location component of $\delta$ to be as great as about any chromosomal length, computed by any method, for which linkage disequilibrium has been observed between any polymorphisms in any population of the species. It is preferable in terms of increasing the power of a version of the invention for linkage studies that the chromosomal location component of $\delta$ be less than about the greatest chromosomal length, computed by any method, for which linkage disequilibrium has been observed between any polymorphisms in any population of the species. In general, the smaller the chromosomal location component of $\delta$, the greater the power of a version of the invention for linkage studies.

It is possible for the frequency distance component of $\delta$ to be as great as about 0.2. ( Depending on the penetrance ratio (r) or the disequilibrium between marker and gene, it is also possible for the frequency distance component of $\delta$ to be greater than 0.2 under some conditions as evidenced by Table 2 under Theory of Operation. So it is also possible for the frequency distance component of $\delta$ to be as great as about 0.25 or higher.) It is preferable in terms of increasing the power of a version of the invention for linkage studies that the frequency distance component of $\delta$ to be less than about 0.2. In general, the smaller the frequency distance component of $\delta$, the greater the power of a version of the invention for linkage studies.

Linkage disequilibrium has been observed between polymorphisms separated by 10 to 12 cM in some homogeneous human populations. Therefore, it is possible for the chromosomal location distance component of $\delta$ to be as large as about 10 to 12 cM, about 10 to 12 million bp, or the equivalent thereof for homogeneous human populations. It is preferable in terms of increasing the power of a version of the invention for linkage studies in human populations that $\delta$ is less than or equal to about [ 1 million bp, 0.15] or the equivalent thereof. It is more preferable in terms of increasing the power of a version of the invention for linkage studies in human populations that $\delta$ is less than or equal to about [ 250,000 bp, 0.1] or the equivalent thereof.

In general, the smaller the magnitude of $\delta$ is in terms of either frequency distance, chromosomal location distance, or both, the greater the power of a version of the invention for linkage studies. In general, the greater N is, the greater the power of a version of the invention for linkage studies. Because the greater N is, the greater the chance that linkage is detected between one or more covering markers and a gene or genes. The largest that N is chosen is limited by the number of known markers in the neighborhood of the CL-F region and also by the distribution of the known markers.

In general, the larger the CL-F region which is N covered, the greater the power of a version of the invention for linkage studies, because a larger region is scanned (covered). Less dense coverings wherein N is small, and the magnitude of $\delta$ is large also have technical and economic advantages for certain situations.

**Specific types of CL-F regions that are N covered**

Specific types of CL-F regions that are N covered are useful. For example, a rectangular CL-F region, a segment-subrange, that is N covered is used in an association based linkage study to test for the presence of a trait causing bi-allelic gene located within the segment-subrange. In the case in which a group of points is N covered to within a CL-F distance [x,y] and the group of points is connected to within a CL-F distance of [2x,2y] or less, then a path connected CL-F region is N covered to within the CL-F distance [x,y].

A CL-F matrix is a device to illustrate and describe the systematic nature of special cases of CL-F regions that are N covered. In the case in which there are N or more markers within each cell of a CL-F matrix, then each point within the matrix is N covered to within the CL-F distance [$L_{CM}$, $W_{CM}$], wherein $L_{CM}$ is the length of a matrix cell and $W_{CM}$ is the width of a matrix cell. A choice of covering markers so that approximately the same number of covering markers are in each cell of a CL-F matrix has utility in that approximately the same amount of effort is expended on each subregion (cell) of the CL-F region defined by the matrix in a linkage study using the covering markers. If the centerpoints of a CL-F matrix (a matrix centerpoint lattice) are each N covered by a group of covering markers to within a CL-F distance [x, y], then each point in the matrix is N covered to within the CL-F distance [2x,2y]. A CL-F matrix can be used as a device to help distinguish versions of the invention from prior art (to the extent that there is prior art).

A requirement that the CL-F region that is N covered to within a certain CL-F distance comprise a certain minimum area or segment-subrange with a certain minimum area is a special case of CL-F regions that are N covered to within the certain CL-F distance. A requirement that the CL-F region that is N covered to within a certain CL-F distance has a certain length or width is a special case of CL-F regions that are N covered to within the certain CL-F distance. Each of these requirements is also a device that can be used to help distinguish versions of the invention from prior art.

**A Note on the Equivalence of Working With individual Alleles of Markers to Perform Two-dimensional Linkage Studies and the CL-F approach using bi-allelic markers**

It is possible to conceptualize performing two-dimensional linkage studies wherein individual marker alleles are used to cover a two-dimensional space, rather than individual bi-allelic markers. Any individual marker allele is assigned a two-dimensional location consisting of the chromosomal location of the marker and the allele frequency of the marker allele. Two-dimensional chromosomal location-allele frequency spaces(or regions) are systematically covered by sets of covering alleles. Each individual covering allele is tested for association with a genetic characteristic. Versions of inventions using systematic chromosomal location-allele frequency(CL-AF) region coverings that are similar to versions of the invention in this application are possible. Indeed these types of inventions have been described in U.S.Provisional Patent Applications previously filed by the inventor.

However, such a conceptual framework and the resulting inventions are equivalent to the CL-F versions approach used in this application. This is because any marker allele, A, that is used as a covering allele can be made to be an allele equivalent of a bi-allelic marker equivalent(BME). So that a BME with allele equivalents A and nonA is a bi-allelic marker with allele A. Therefore, any set of covering alleles that systematically cover a two-dimensional CL-AF region is equivalent to a set of BMEs that systematically cover an equivalent CL-F region. Testing each covering allele for association with a genetic characteristic is exactly equivalent to testing each BME of a set of BMEs for evidence of linkage to a gene using a statistical linkage test based on allelic association. Even testing for the presence or absence of a covering allele in the chromosomal DNA of an individual is equivalent to genotyping the individual at a BME. And determining a sample allele frequency for a covering allele, is equivalent to determining the sample allele frequencies for a BME.

**Example 1 of Process #1 is used for identifying markers linked to a disease gene.**

Example 1 A process for identifying bi-allelic markers linked to a bi-allelic disease gene in human beings, comprising the steps of :

a)choosing two or more bi-allelic covering markers so that a CL-F region is N covered to within a CL-F distance [250,000 bp, 0.1] or the equivalent thereof by the covering markers, wherein N is an integer number greater than or equal to 2 ;

b)choosing the same statistical linkage test based on allelic association for each covering marker;

c)choosing the same sample of individual human beings for each covering marker;

d)obtaining genotype data at each covering marker for each individual in the sample and obtaining phenotype status data for the disease for each individual in the sample ;

e)calculating evidence for linkage between each covering marker and the gene using the test chosen in step b) and the genotype data at each covering marker and the using the phenotype status data for the disease for each individual in the sample ; and

f)identifying those covering markers as linked to the gene which show evidence for linkage based on the calculations of step e.

**Apparatus Versions**

**General step by step descriptions of individual apparatus versions are given below.**

**Apparatus #1, an apparatus to practice process #1.**

**Apparatus #1,** An apparatus for identifying bi-allelic markers linked to a bi-allelic genetic characteristic gene in a species of creatures, comprising:

a)means for choosing two or more bi-allelic covering markers so that a CL-F region is systematically covered by the two or more covering markers;

b)means for choosing a statistical linkage test based on allelic association for each covering marker;

c)means for choosing a sample of individuals for each covering marker ;

d)means for obtaining genotype data/sample allele frequency data for each covering marker and the sample chosen for each covering marker, and for obtaining phenotype status data for the genetic characteristic for each individual in the sample chosen for each covering marker;

e)means for calculating evidence for linkage between each covering marker and the gene using the statistical linkage test based on allelic association chosen for each covering marker and the genotype data/sample allele frequency data for each covering marker and using the phenotype status data for the genetic characteristic for each individual in the sample chosen for each covering marker obtained in d); and

f)means for identifying those covering markers as linked to the gene which show evidence for linkage based on the calculations by means e).

**More detailed description of Apparatus #1:** Apparatus #1 is an apparatus to practice process #1. More details of the description of apparatus #1 are found under the description of Process #1 above. Any one of the means labeled a), b), c), d), e) or f) of apparatus #1 includes any means for automating or partially automating a step as step a), b), c), d), e) or f) respectively of process #1. An example of any one of the means in this paragraph labeled a), b), c), d), e), or f) is means comprising an appropriately programmed, suitable computer, the computer being supplied with proper data and instructions.

The means labeled d) of apparatus #1 for obtaining genotype data/ sample allele frequency data for each covering marker for the sample chosen for each covering marker includes any automated or partially automated means to obtain genotype data/ sample allele frequency data. An example of means to obtain genotype data/ sample allele frequency data is means using mass spectrometry.' Means to obtain genotype data/ sample allele frequency data that is automated or partially automated includes means comprising Oligonucleotide Technology described below.

**Apparatus #1A, an apparatus to practice process #1A.**

**Apparatus#1A: An apparatus for localizing a bi-allelic genetic characteristic gene in a species of creatures to a chromosomal location-least common allele frequency (CL-F) region, comprising the means a), b), c), d) and e) of Apparatus #1 and further comprising the means of:**

**f)means for localizing the gene to the approximate chromosomal location-least common allele frequency region (CL-F)of one or more markers that show evidence for linkage based on the calculations of means e).**

An example of means f) is means comprising an appropriately programmed, suitable computer, the computer being supplied with proper data and instructions. Further details of this apparatus which practices process #1A are under process #1 and process #1A and **Software**(above).

**Genotype data/Sample allele frequency data apparatus**

An apparatus to obtain genotype data/sample allele frequency data similar to the data of the step d) of process #1 has great utility in that it is used to provide genotype data /sample allele frequency data for the more powerful two-dimensional linkage studies introduced in this application.

**ApparatusGd/Safd#1: Genotype data/Sample allele frequency data apparatus: An apparatus for obtaining genotype data/sample allele frequency data for each bi-allelic marker of a group of two or more bi-allelic covering markers in the chromosomal DNA of one or more individuals of a sample, comprising:**

**a) means for determining information on the presence or absence of each allele of each bi-allelic marker of a group of two or more bi-allelic covering markers in the chromosomal DNA of one or more individuals of the sample, a CL-F region being systematically covered by the two or more bi-allelic covering markers; and**

**b) means for transforming the information of step a) into genotype data/sample allele frequency data for each marker of the group.**

The CL-F region and covering markers are for a species and the one or more individuals are members of the species. Means for determining information on the presence or absence of each allele of each bi-allelic marker of the group in chromosomal DNA includes any means of determination. Means for determining information on the presence or absence of each allele of each bi-allelic marker of the group in chromosomal DNA includes means comprising oligonucleotide technology by using a set of oligonucleotides that is complementary to the group as discussed below. Information on the presence or absence of each allele in the chromosomal DNA is obtained using a DNA specimen from each of one or more individuals of the sample or by using one or more DNA pools of DNA specimens from two or more individuals of the sample. Any apparatus that obtains genotype data or sample allele frequency data (similar to the data of the step d) of process #1) by determining the presence or absence of each allele of each bi-allelic marker of the group in the chromosomal DNA of one or more individuals is an example of this version of the invention. Versions of this apparatus also obtain a combination of genotype data and sample allele frequency data similar to the data of the step d) of process #1. The details of step b) will be clear to those of ordinary skill in the art.

Each bi-allelic covering marker is a true bi-allelic or BME. Determining the presence or absence of each allele of each bi-allelic marker in the group includes determining the presence or absence of each allele equivalent of each bi-allelic marker equivalent(BME) in the group. Any method of systematically covering the CL-F region is acceptable. In this application, the systematic covering of a CL-F region in versions of the invention is described mathematically as the covering of a CL-F region, wherein the CL-F region is N covered to within a CL-F distance $\delta$ by two or more bi-allelic covering markers. For further details regarding this, see <u>Detailed Description of the Systematic Covering of a CL-F Region Used In Versions of the Invention</u> above.

An example of ApparatusGd/Safd#1Genotype data/Sample allele frequency data apparatus, a sample allele frequency apparatus:

Example 1 of ApparatusGd/Safd#1:An apparatus for obtaining genotype data/sample allele frequency data for each bi-allelic marker of a group of two or more bi-allelic covering markers in the chromosomal DNA of one or more individuals of a sample, wherein the genotype data/sample allele frequency data is sample allele frequency data, comprising:

a) means for determining information on the presence or absence of each allele of each bi-allelic marker of a group of two or more bi-allelic covering markers in the chromosomal DNA from one or more individuals of the sample, a CL-F region being N covered to within the CL-F distance [ 1.0 cM, 0.15] by the two or more bi-allelic covering markers, wherein N is an integer number greater than or equal to 1; and

b) means for transforming the information of step a) into sample allele frequency data for each marker of the group.

Example 2 of ApparatusGd/Safd#1:An apparatus for obtaining genotype data/sample allele frequency data for each bi-allelic marker of a group of two or more bi-allelic covering markers in the chromosomal DNA of an individual, wherein the genotype data/sample allele frequency data is genotype data, comprising:

a) means for determining information on the presence or absence of each allele of each bi-allelic marker of a group of two or more bi-allelic covering markers in the chromosomal DNA from an individual, a CL-F region being N covered to within the CL-F distance [12cM, 0.25] or the equivalent thereof by the two or more bi-allelic covering markers, wherein N is an integer number greater than or equal to 1; and

b) means for transforming the information of step a) into genotype data for each marker of the group. (It should be noted that the following genotype apparatus is equivalent to Example 2 of ApparatusGd/Safd#1: Genotype Apparatus: An apparatus for genotyping an individual, comprising:

a)means to genotype an individual at two or more bi-allelic covering markers, a CL-F region being N covered to within the CL-F distance [12cM, 0.25] or the equivalent thereof by the two or more bi-allelic covering markers, wherein N is an integer number greater than or equal to 1. )

**Genotype data/Sample allele frequency data process**

A process to obtain genotype data/sample allele frequency data similar to the data of the step d) of process #1 has great utility in that it is used to provide genotype data /sample allele frequency data for the more powerful two-dimensional linkage studies introduced in this application.

**Description of the Genotype data/Sample allele frequency data process.**

**ProcessGd/Safd#1: Genotype data/Sample allele frequency data process: A process for obtaining genotype data/sample allele frequency data for each bi-ailelic marker of a group of two or more bi-allelic covering markers in the chromosomal DNA of one or more individuals of a sample, comprising:**

**a) determining information on the presence or absence of each allele of each bi-allelic marker of a group of two or more bi-allelic covering markers in the chromosomal DNA of one or more individuals of the sample, a CL-F region being systematically covered by the two or more bi-allelic covering markers; and**

**b) transforming the information of step a) into genotype data/sample allele frequency data for each marker of the group.**

The CL-F region and covering markers are for a species and the one or more individuals are members of the species. Determining information on the presence or absence of each allele of each bi-allelic marker of the group in chromosomal DNA includes any method of determination. Determining information on the presence or absence of each allele of each bi-allelic marker of the group in chromosomal DNA includes methods comprising oligonucleotide technology by using a set of oligonucleotides that is complementary to the group as discussed below. Information on the presence or absence of each allele in the chromosomal DNA is obtained using a DNA specimen from each of one or more individuals of the sample or by using one or more DNA pools of DNA specimens from two or more individuals of the sample. Any process that obtains genotype data or sample allele frequency data (similar to the data of the step d) of process #1) by determining the presence or absence of each allele of each bi-allelic marker of the group in the chromosomal DNA of one or more individuals is an example of this version of the invention. Versions of this process also obtain a combination of genotype data and sample allele frequency data similar to the data of the step d) of process #1. The details of step b) will be clear to those of ordinary skill in the art.

Each bi-allelic covering marker is a true bi-allelic or BME. Determining the presence or absence of each allele of each bi-allelic marker in the group includes determining the presence or absence of each allele equivalent of each bi-allelic marker equivalent(BME) in the group. Any method of systematically covering the CL-F region is acceptable. In this application, the systematic covering of a CL-F region in versions of the invention is described mathematically as the covering of a CL-F region, wherein the CL-F region is N covered to within a CL-F distance $\delta$ by two or more bi-allelic covering markers. For further details regarding this, see Detailed Description of the Systematic Covering of a CL-F Region Used In Versions of the Invention above.

**An example of ProcessGd/Safd#1Genotype data/Sample allele frequency data process, a genotype data process:**

Example 1 of ProcessGd/Safd#1:A process for obtaining genotype data/sample allele frequency data for each bi-allelic marker of a group of two or more bi-allelic covering markers in the chromosomal DNA of an individual, wherein the genotype data/sample allele frequency data is genotype data, comprising:

a) determining information on the presence or absence of each allele of each bi-allelic marker of a group of two or more bi-allelic covering markers in the chromosomal DNA from an individual, a CL-F region being N covered to within the CL-F distance [12cM, 0.25] or the equivalent thereof by the two or more bi-allelic covering markers; wherein N is an integer number greater than or equal to 1; and

b) transforming the information of step a) into genotype data for each marker of the group. (It should be noted that the following genotype process is equivalent to Example 1 of ProcessGd/Safd#1: Genotype Process: A process for genotyping an individual, comprising:

a) genotyping an individual at two or more bi-allelic covering markers, a CL-F region being N covered to within the CL-F distance [12cM, 0.25] or the equivalent thereof by the two or more bi-allelic covering markers, wherein N is an integer number greater than or equal to 1. )

**Oligonucleotide technology**

Each version of oligonucleotide technology is a means to sense the presence or absence of each of one or more true alleles of a group of true alleles in chromosomal DNA from one or more individuals by means of a hybridization reaction with an oligonucleotide that is complementary to each of the one or more true alleles (see definitions section). Thus versions of oligonucleotide technology are a means of genotyping one or more individuals. And, versions of oligonucleotide technology are a means of obtaining sample allele frequency data for one or more marker alleles for a sample of individuals using pooled DNA from the individuals in the sample.

In Some Versions of Oligonucleotide Technology for Genotyping or Obtaining Sample Allele Frequency Data, a Physico-chemical Signal is Generated when an Allele in Chromosomal DNA and a Complementary Oligonucleotide Hybridize

Some versions of oligonucleotide technology for genotyping or for obtaining sample allele frequency data use a sensor which includes one or more oligonucleotides which are complementary to an allele. When the sensor is exposed to chromosomal DNA from an individual who carries the allele, the oligonucleotides which are complementary to the allele hybridize with chromosomal DNA specimens of the allele. The hybridization generates a physico-chemical signal which indicates the presence of the allele in the chromosomal DNA of the individual. The lack of the physico-chemical signal indicates no (or negligible)hybridization and that the allele is not present in the chromosomal DNA of an individual. Examples of oligonucleotide technology for genotyping, obtaining sample allele frequency data or genotype data/ sample allele frequency data

Companies like Affymetrix are using high density arrays of oligonucleotides attached to silicon chips or glass slides to genotype DNA from one individual at thousands of bi-allelic markers.[VIII] In some of these versions of oligonucleotide technology, the strength of hybridization of oligonucleotides that differ at only one base to DNA containing an SNPare compared to determine genotype.[IX] Another version of oligonucleotide technology uses oligonucleotides as PCR (Polymerase Chain Reaction) primers to obtain genotype data.[X]Other examples of oligonucleotide technology and it's uses to obtain genetic information are included in the articles cited in the endnotes.[XI] Versions of oligonucleotide technology obtain sample allele frequency data from pooled DNA or genotype data using oligonucleotides as PCR primers to obtain amplified reaction products that are detected by mass spectrometry. Another example of oligonucleotide technology is padlock probes.[XII]

Other examples of oligonucleotide technology are minisequencing on DNA arrays, dynamic allele-specific hybridization, microplate array diagonal gel electrophoresis, pyrosequencing, oligonucleotide-specific ligation, the Taq-Man system and immobilized padlock probes as presented at the First International Meeting on Single Nucleotide Polymorphism and Complex Genome Analysis.[XIII]

Sets of Oligonucleotides for Genotyping at Bi-allelic Markers or Obtaining Sample Allele Frequency Data

*A set of oligonucleotides that is complementary(see definitions) to a group of one or more bi-allelic markers has utility to determine genotype data at each of the markers in the group*, including groups with BMEs and approximately bi-allelic markers.

Similarly, a set of oligonucleotides that is complementary to a group of bi-allelic markers has utility to obtain sample allele frequency data for each allele of each marker in the group.

*In both* cases, *obtaining genotype data or sample allele frequency* data, the same *principle is used: a set of oligonucleotides* that is *complementary* to a *group* of *bi-allelic markers has utility determine the* presence or absence *of each* allele of each *marker in the group in chromosomal DNA.*

**Using sets of oligonucleotides to obtain Genotype Data/Sample Allele Frequency Data for each marker of a group of bi-allelic markers, wherein the group of markers systematically cover a CL-F region**

Genotype data/sample allele frequency data for each marker of a group of bi-allelic markers, wherein the group of bi-allelic markers systemically cover a CL-F region has great utility for use in the more powerful two-dimensional linkage studies introduced in this application. As described above under Oligonucleotide Technology, some sets of oligonucleotides have utility to determine genotype data at each bi-allelic marker of a group of one or more bi-allelic markers. Similarly, some sets of oligonucleotides have utility to obtain sample allele frequency data for each bi-allelic marker of a group of one or more bi-allelic markers. Therefore, the use of one or more copies of a set of oligonucleotides to obtain genotype data or sample allele frequency data for each bi-allelic marker of a group of one or more bi-allelic covering markers, wherein the group of bi-allelic covering markers systematically cover a CL-F region has great utility.

A word to avoid confusion in terminology: in this application, a set of markers for use in genotyping is referred to as a set of oligonucleotides.

A set of oligonucleotides consisting of one or both strands of each allele of a group of one or more markers is a set of oligonucleotides that is complementary to the group of markers. (see definitions section) Such a set of oligonucleotides is in effect the group of markers themselves; and such a set of oligonucleotides has utility to determine genotype data at each marker in the group. So a group of markers (or set of markers) for use in obtaining genotype data or sample allele frequency data for each of the markers in the group is included in the descriptive phrase: "a set of oligonudeotides".

**Description of Use set#1 D:**

**Use set#1 D The use of one or more copies of a set of oligonucleotides to determine genotype data/ sample allele frequency data for each bi-allelic marker of a group of two or more bi-allelic covering markers for one or more individuals, wherein the group of covering markers systematically cover a CL-F region.**
The CL-F region and covering markers are for a species and the one or more individuals are members of the species. An example of a set of oligonucleotides with utility to be used to determine genotype data/sample allele frequency data for each bi-allelic marker of a group of two or more bi-allelic covering markers is a set of oligonucleotides that is complementary to the group of markers. A set that is complementary to the group of markers is used to detect the presence or absence of each the alleles of the covering markers by means of a hybridization reaction as discussed under oligonucleotide technology. Thus a set that is complementary to the group of markers is used to determine genotype data/sample allele frequency data for each covering marker.
The use of one or more copies of a set of oligonucleotides to obtain genotype data or sample allele frequency data for each bi-allelic marker of a group of one or more bi-allelic covering markers, wherein the group of bi-allelic covering markers systematically cover a CL-F region are both examples of this version of the invention(Use Set#1D).
In this application, the systematic covering of a CL-F region in versions of the invention is described mathematically as the covering of a CL-F region, wherein the CL-F region is N covered to within a CL-F distance $\delta$ by two or more bi-allelic covering markers. For further details regarding this, see <u>Detailed Description of the Systematic Covering of a CL-F Region Used in Versions of the Invention</u> above. **<u>Example 15 of Use set#1D:</u>** The use in genotyping one or more individuals, of one or more copies of a set of oligonucleotides, the set of oligonucleotides being complementary to a group of two or more bi-allelic covering markers, a CL-F region being N covered by the covering markers to within a CL-F distance of about [ 250,000 bp, 0.1] or the equivalent thereof, wherein N is an integer greater than or equal to two.

**Composition of matter: Description of Comp set#1D:**

**Comp set#1D: One or more copies of a set of oligonucleotides, the set of oligonucleotides being complementary to a group of two or more bi-allelic covering markers, wherein the group of covering markers systematically cover a CL-F region.**
A set of oligonucleotides that is complementary to a group of two or more bi-allelic covering markers, wherein the group of covering markers systematically cover a CL-F region has great utility for use in the two-dimensional linkage study techniques introduced in this application. Such a set has utility in being used to genotype individuals or obtain sample allele frequency data or genotype data/sample allele frequency data as described above under Use set#1 D. In this application, the systematic covering of a CL-F region in versions of the invention is described mathematically as the covering of a CL-F region, wherein the CL-F region is N covered to within a CL-F distance $\delta$ by two or more bi-allelic covering markers. For further details regarding this, see <u>Detailed Description of the Systematic Covering of a CL-F Region Used in Versions of the Invention</u> above.

**<u>Example 1Comp of Comp set#1D:</u>**

**<u>Example 1Comp:</u>**One or more copies of a set of oligonucleotides, the set of oligonucleotides being complementary to a group of two or more bi-allelic covering markers, a CL-F region being N covered by the covering markers to within a CL-F distance of about [1cM, 0.2] or the equivalent thereof, wherein N is an integer greater than or equal to one.

**<u>Redundancy of Covering Markers</u>**

Some versions of the invention make use of N coverings of CL-F regions by covering markers which limit (possibly to zero) the number of pairs of covering markers which are redundant within CL-F distance D, D = [$D_{CL}$, $D_F$], wherein D is less than or equal to about $\delta$, a CL-F covering distance. This limits the number covering markers which are separated by a CL-F distance of less than or equal to D(if the markers were placed on a CL-F map) which *will be in extreme positive disequilibrium with each other*. This limitation is done by requiring that less than or equal to R pairs of covering markers are redundant within distance D. Wherein R is an integer greater than or equal to 0 and less than or equal to about N(N-1)/2. When R is chosen to be zero, no pair of covering markers is redundant within distance D.
A preferable condition is that each bi-allelic covering marker within each small CL-F region (a small segment-subrange of length about $\delta_{CL}$ and width about $\delta_F$ the distance components of the covering distance $\delta$ ) provides much new (i.e. non-redundant) information about linkage and association to any nearby bi-allelic gene. Under these conditions, testing each bi-allelic covering marker in each small CL-F region increases the likelihood of detecting linkage to a gene.
Limiting (including to zero) pairs of covering markers which are redundant within CL-F distance D(which is less than or equal to a covering distance $\delta$ ) approaches and achieves this preferable condition. This limitation is not crucial to the functioning of a version of the invention, however, it has the advantage of reducing excess effort and increasing efficiency.

**Polymorphism CL-F Display**

Polymorphism CL-F display apparatus display the chromosomal location, least common allele frequency and identity of each polymorphism of one or more polymorphisms (markers or genes or both) of one or more populations of one or more species on one or more two-dimensional graphs, each graph is similar to an x-y plot. The apparatus has utility including aiding in decisions regarding linkage studies and the interpretation of linkage study data.

The apparatus comprise means to display the chromosomal location, least common allele frequency and identity of each polymorphism of one or more polymorphisms (markers or genes or both) of one or more populations of one or more species on one or more two-dimensional graphs, each graph is similar to an x-y plot.

Each graph has two axes, one axis, the frequency axis, represents least common allele frequency and the altemate(or other) axis, the chromosomal location axis, represents chromosomal location. Each frequency axis of each graph is in units of population frequency. Each chromosomal location axis of each graph is in units of chromosomal location such as centimorgans, base pairs or the equivalent thereof.

The frequency axis of each graph spans the entire range 0 to 0.5 or a subrange of the range 0 to 0.5. The chromosomal location axis of each graph spans the chromosomal locations on one or more segments of one or more chromosomes of a species, each of the one or more segments is a size from the equivalent of a base pair in length to the length of an entire chromosome (or the equivalent thereof). Each point on each graph is directly opposite a value on the frequency axis of each graph. The value on the frequency axis directly opposite each point on each graph is the frequency coordinate of each point on each graph. Each point on each graph is directly opposite a value on the chromosomal location axis of each graph. The value on the chromosomal location axis directly opposite each point on each graph is the chromosomal location coordinate of each point on each graph.

Each graph displays the chromosomal location and least common allele frequency of each polymorphism of one or more polymorphisms. Each polymorphism displayed on each graph is assigned a graph location on each graph.

The graph location of each polymorphism displayed on each graph is typical of the use of x-y plots. The graph location assigned to each polymorphism on each graph is a point. The chromosomal location coordinate of the point assigned as the graph location to any one polymorphism is equal (or approximately equal) to the chromosomal location of the polymorphism. And the frequency coordinate of the point assigned as the graph location to any one polymorphism is equal (or approximately equal) to the least common allele frequency of the polymorphism.

The apparatus comprise means for displaying one or more two-dimensional graphs. Each graph comprises, the identity and graph location of one or more polymorphisms assigned a location on each graph. And the apparatus comprise means for displaying one or more graphs wherein the viewer chooses the species, population, polymorphisms, span of the frequency axis and span of the chromosomal location axis of the one or more graphs ; in versions of the apparatus, the means of this sentence comprises a computer.

The apparatus comprise means for storing and updating data on the chromosomal location and least common allele frequency of one or more polymorphisms of one or more populations of one or more species and means for storing chromosomal location and least common allele frequency data on newly discovered polymorphisms.

Versions of the apparatus comprise means for printing each of the one or more graphs.

**Theory of Operation I Best Mode**

Systematically Varying Both Marker Chromosomal Location and Marker Allele Frequency of Markers_in Linkage Studies

The inventor's calculations and observations have demonstrated the increased power of the TDT in more common, less optimal situations when a bi-allelic marker and bi-allelic gene have (1) similar but not identical allele frequencies and (2) the marker and gene are in some degree of linkage disequilibrium. Thus, for a typical linkage study using bi- allelic markers and an association based linkage test, ***to increase the likelihood of both criteria (1) and (2) occurring for one or more markers,* so as to *increase the power of an association based linkage test in* a *linkage study, the bi-allelic markers used in the study are chosen so that the least common allele frequencies of the markers vary systematically over a range or subrange of least common allele frequency AND the chromosomal location of the markers vary systematically over one or more chromosomes or chromosomal regions. And the bi-allelic markers are chosen* so *that the markers' chromosomal locations and least common allele frequencies vary systematically in an essentially independent manner.***

(In theTheory of Operation/ Best Mode Section the traditional symbol used in scientific papers for the disequilibrium coefficient, $\delta$, is used. This should not be confused with the symbol $\delta$ used for the covering distance in the remainder of the application. The symbol d is used for the disequilibrium coefficient in the sections of the application other than the Theory of Operation/Best Mode Section.) The theory of operation is based on the mathematical observation that the TDT and other association-based tests for linkage are increased in power as the frequencies of the disease-causing allele of a bi-allelic gene and the positively associated allele of a linked bi-allelic marker become similar in magnitude. The inventor made this observation as a result of deriving the equation shown below for $P_t$ (this is Equation 2 in the unpublished manuscript submitted for publication in December 1996 and in published paper by RE McGinnis in the Annals of Human Genetics vol 62, pp. 159-179, 1998).

$$P_t = .5 + (1 - 2\theta)\left[\frac{c_1 c_4 - c_2 c_3}{H}\right]\left\{p^2\left(\frac{\alpha^2 - \beta^2}{4}\right) + 2p(1-p)\left(\frac{(\alpha+\beta)^2 - (\beta+\gamma)^2}{16}\right) + (1-p)^2\left(\frac{\beta^2 - \gamma^2}{4}\right)\right\}$$

Equation 2

$P_t$ may be regarded as the size of the "signal" which is given by the TDT to indicate that a tested marker is linked to a disease-causing gene. The more $P_t$ is elevated above 0.5 (baseline), the greater is the evidence for linkage or "power" provided by the association-based linkage test known as the TDT.

Table 2 in the unpublished manuscript filed with previous US Provisional Patent Applications(see below) illustrates how signal strength increases substantially as the frequencies of disease-causing allele and positively associated marker allele become similar in magnitude. As noted on pages 24 and 25 of the unpublished manuscript(see below), Table 2 assumes that the frequency (p) of the disease-causing allele is fixed at p=.1 while the frequency (m) of the positively associated marker, allele varies (m=.5, .3, .2, .1, .05). Note that when the level of disequilibrium (or association) between the bi-allelic marker and bi-allelic disease gene is fixed (in this case either $\delta=\delta_{max}$ or $\delta=\frac{1}{2}\delta_{max}$), the signal strength of $P_t$ progressively increases as m decreases from m=.5 to m=.1 (the same frequency as the disease allele, i.e., p=.1). For example, in the section of Table 2 for r=5, note that when $\delta=\frac{1}{2}$

$\delta_{max}$, $P_t$ is .548 at m=.5 and then steadily increases to .572 (m=3), .597 (m=.2), 648 (m=.1) and then starts to decrease again as m departs from m=p=.1 (i.e. $P_t$=.636 at m=.05). As noted on pages 24-25 (below)of the unpublished manuscript, the TDT chi-square statistic (assuming a sample size of 200 families) is such that the signal strength at m=. 5 ($P_t$=.548) does not produce a statistically significant evidence for linkage (p-value > 0.05) while the doubling of signal strength at m=.2 ($P_t$=.597) produces very strong statistical evidence for linkage by the TDT (p-value< 0.005). This sort of substantial increase in power is also true of other association-based linkage tests as the frequencies of the disease-causing allele and associated marker allele become more similar in magnitude.

Table 2(Footnotes for Table 2 are on next page)

Effect of penetrance ratio (r), disequilibrium (δ) and marker heterozygosity (m) on magnitude of $P_t$ and $P_S$

| | | Magnitude of $P_t$ | | | Magnitude of $P_S$ | | |
|---|---|---|---|---|---|---|---|
| | | $\delta_{max}$[a] | $\frac{1}{2}\delta_{max}$[b] | δ=0 | $\delta_{max}$[a] | $\frac{1}{2}\delta_{max}$[b] | δ=0 |
| r=2 | m=.5 | .526 | .513 | .500 | .505 | .505 | .504 |
| | m=.3 | .541 | .521 | .500 | .508 | .506 | .504 |
| | m=.2 | .558 | .531 | .500 | .511 | .508 | .504 |
| | m=.1 | .595 | .555 | .500 | .518 | .512 | .504 |
| | m=.05 | .589 | .552 | .500 | .517 | .511 | .504 |
| r=5 | m=.5 | .596 | .548 | .500 | .543 | .540 | .539 |
| | m=.3 | .633 | .572 | .500 | .561 | .548 | .539 |
| | m=.2 | .666 | .597 | .500 | .575 | .556 | .539 |
| | m=.1 | .719 | .648 | .500 | .600 | .573 | .539 |
| | m=.05 | .696 | .636 | .500 | .589 | .571 | .539 |
| r=10 | m=.5 | .656 | .577 | .500 | .595 | .587 | .584 |
| | m=.3 | .702 | .612 | .500 | .623 | .600 | .584 |

(continued)

Effect of penetrance ratio (r), disequilibrium ($\delta$) and marker heterozygosity (m) on magnitude of $P_t$ and $P_S$

| | | Magnitude of $P_t$ | | | Magnitude of $P_S$ | | |
|---|---|---|---|---|---|---|---|
| | m=.2 | .736 | .644 | .500 | .644 | .612 | .584 |
| | m=.1 | .785 | .703 | .500 | .673 | .635 | .584 |
| | m=.05 | .750 | .684 | .500 | .652 | .628 | .584 |
| r=∞ | m=.5 | .740 | .617 | .500 | .700 | .680 | .673 |
| | m=.3 | .791 | .663 | .500 | .743 | .700 | .673 |
| | m=.2 | .826 | .703 | .500 | .772 | .716 | .673 |
| | m=.1 | .870 | .770 | .500 | .807 | .744 | .673 |
| | m=.05 | .816 | .741 | .500 | .763 | .730 | .673 |

Footnotes for Table 2

[a,b]value of $\delta$ that is maximal ($\delta_{max}$) and half-maximal $(\frac{1}{2}\delta_{max})$, as determined by the heterozygosity of the marker (m) and disease locus (p=.1)

Importance of disequilibrium and marker heterozygosity (i.e. marker allele frequency) in detecting linkage

When the heterozygosity (i.e. allele frequencies) of a bi-allelic marker and bi-allelic disease locus are fixed, ($P_S$ - .5) and $|P_t$ - .5| are both maximized at the most positive or most negative possible value of $\delta$ ($\delta_{max}$, $\delta_{min}$), as demonstrated in the published paper. This maximization of $\chi^2_{asp}$ and $\chi^2_{tdt}$ is intimately connected to $M_S$ and Mt (defined in equations I and 2) since: (a) these are the only two factors in $P_S$ and $P_t$ that are influenced by $\delta$ and (b) $M_S$ and $|M_t|$ are maximal and equal to each other when $\delta$ is extreme ($\delta_{max}$ or $\delta_{min}$). Furthermore, as explained in the published paper, $M_S$ is a measure of the proportion of informative (A/B) parents who are also informative (D/d) at the disease locus. Therefore, maximizing $M_S$ (and, by implication, $|M_t|$) is equivalent to *minimizing* the proportion of A/B parents who are homozygous (D/D or d/d) at the disease locus. Such homozygous D/D or d/d parents contribute evidence *against* linkage since they transmit marker alleles A and B to affected offspring with equal probability; thus, minimizing their proportion among A/B parents being tested for linkage has the effect of maximizing $\chi^2_{asp}$ and $\chi^2_{tdt}$.

Nevertheless, when bi-allelic markers have a specific (i.e. fixed) heterozygosity different from that of a bi-allelic disease locus, some A/B parents must be homozygous at the disease locus, even when $\delta$ is extreme. But if marker heterozygosity is variable, the proportion of A/B parents who are D/D or d/d approaches *zero* as marker heterozygosity approaches that of the disease locus and as $\delta$ approaches $\delta_{max}$ or $\delta_{min}$. Consequently, the most extreme values of $P_t$ and $P_S$, and highest values of $\chi^2_{tdt}$ and $\chi^2_{asp}$ are found when marker and disease locus have equal heterozygosity and $\delta=\delta_{max}$ or $\delta=\delta_{min}$.

Example illustrating the importance of marker heterozygosity (i.e. allele frequency)

To illustrate the importance of marker heterozygosity and disequilibrium, Table 2 shows $P_t$ and $P_S$ values when the frequency (p) of disease allele D is constant at 0.1, but the frequency (m) of marker allele A varies between m=.5 (maximum marker heterozygosity) and m=.1 (equal heterozygosity at marker and disease loci). The table assumes mode of inheritance is additive, and separate sections of the table show the results when the penetrance ratio (r) is 2, 5, 10 or ∞. For each value of r, an individual line in the table represents constant marker heterozygosity (m=.5, .3, .2, or .1) and from left-to-right on each line, one sees $P_t$ and $P_S$ values when $\delta=\delta_{max}$, $\delta=\frac{1}{2}\delta_{max}$, and $\delta=0$, the value of $\delta_{max}$ being determined by the particular values of m and p [$\delta_{max}=p(1-m)$]. As noted in Appendix 1 of the published paper, when p<m and p<(1-m), as in this example, the most extreme values of $P_t$ and $P_S$ must occur at $\delta=\delta_{max}$. This can be seen in each line of the table by the steady increase in both $P_t$ and $P_S$ as one moves from $\delta=0$ to $\delta=\delta_{max}$, with every line also showing $P_t > P_S$ at $\delta=\delta_{max}$ and most lines showing $P_t > P_S$ at $\delta=\frac{1}{2}\delta_{max}$.

Most remarkable, however, are the sizeable increases in $P_S$ and even greater increases in Pt as marker heterozygosity drops toward the heterozygosity of the disease locus (m→.1). A typical example is at r=5 and $\delta=\frac{1}{2}\delta_{max}$

where the table shows $P_t$=.548 at maximum marker heterozygosity (m=.5) and $P_t$=.597 or .648 for m=.2 or .1, respectively. The impact of such an increase in Pt can be understood by calculating $\chi^2_{tdt}$ for $P_t$=.548 (m=.5) and for $P_t$=.597 (m=.2) assuming a data set of 200 families each with two affected sibs. Based on the expression for $\dfrac{H}{F}$ , I calculate the proportion of A/B parents to be .50 and .39 when m=.5 and .2, respectively. So for m=.5, there would be .5 x 400 x 2 = 400 informative transmissions to affected offspring with transmissions of allele A totaling .548 x 400 = 219, thus implying

$$\chi^2_{tdt}=\frac{38^2}{400} = 3.61,$$ p<0.1. For m=.2, there would be .39 x 400 x 2 = 312 informative transmissions of which .597 x 312 = 186 would be transmissions of allele A yielding $$\chi^2_{tdt}=\frac{60^2}{312} =11.54,$$ p<0.005.

This example is typical, and highlights perhaps the most important finding of this paper; namely the importance of using bi-allelic markers with heterozygosity similar to that of a bi-allelic disease locus. Indeed, since a majority of susceptibility loci may be bi-allelic, the judicious use of bi-allelic markers of both high, medium, and low heterozygosity may be crucial in order to initially detect and replicate linkages to loci conferring modest disease risk.

### Best Mode:

Method for locating disease causing polymorphism using biallelic linkage analysis

Objective :To test, by association-based linkage analysis (e.g., by TDT), whether a disease-causing polymorphism is located on a particular chromosome (e.g., human chromosome 4) or within a particular subregion of that chromosome.

PART 1 - Steps in conducting the association-based linkage test

Step 1

To conduct the test, first divide the chromosome or subregion of interest into segments that are short enough that polymorphisms within each segment are likely to be in linkage disequilibrium with each other. The division of a chromosome or subregion of interest into "segments" is conceptual (not physical) and is based on chromosomal maps such as those provided by the Whitehead Institute or Marshfield Foundation for Biomedical Research. Although disequilibrium has been observed in Finnish populations between polymorphisms that are 7 to 10 centimorgans (cM) apart, the chromosomal segments for searching for disease-causing polymorphisms in more genetically heterogeneous populations should be less than 1 cM long (e.g., 250,000 base pairs long). These chromosomal segments might or might not overlap each other (i.e., share some of their length in common); but the set of chromosomal segments should completely cover the entire chromosome or entire subregion of interest, so that a disease-causing polymorphism located anywhere on the chromosome or anywhere in the subregion of interest will be detected by the test.

Step 2

It is well known that increased disequilibrium between a marker and linked disease locus increases evidence for linkage provided by association-based linkage tests such as the TDT. However, what has not been recognized is that the specific allele frequencies of the marker locus can also have an enormous impact on the strength of evidence for linkage. I showed this by analyzing equation 2 for $P_t$. Specifically, when a bi-allelic marker is in linkage disequilibrium with a bi-allelic disease locus, the strength of evidence for linkage provided by the TDT is *greatly* increased if the bi-allelic marker and bi-allelic disease locus have similar allele frequencies.

This phenomenon is illustrated by Table 2 and explained above. For example, suppose as noted above, that the susceptibility allele ("allele D") of a bi-allelic disease locus has a frequency of 0.1 and further suppose that the disease locus is in half-maximal positive disequilibrium with a bi-allelic marker $(\delta=\frac{1}{2}\delta_{max})$. As noted above, $\chi^2_{tdt}$ will equal only 3.61 (p< 0.1) if the frequency of the less common marker allele is 0.5; but if the frequency of the less common marker allele is 0.2 (and hence much closer to the frequency of allele D) then $\chi^2_{tdt}$ will equal 11.54, thus providing much stronger evidence for linkage (p<0.005).

*Therefore*, in searching for association-based linkage to a bi-allelic disease locus within each of the aforementioned chromosomal segments (see step 1), it is crucial to identify and test (e.g., by TDT) bi-allelic markers within each segment that have a broad range of allele frequencies. An unidentified bi-allelic disease locus could have allele frequencies close to 0.5/0.5, 0.4/0.6, 0.3/0.7, 0.2/0.8, 0.1/0.9 or below 0.1/above 0.9; hence, it is crucial to test bi-allelic markers with frequencies near 0.5/0.5 and near 0.1/0.9 as well as test others with allele frequencies that fall at regular increments between the extremes of 0.5/0.5 and 0.1/0.9. By testing bi-allelic markers with a broad range of allele frequencies that are spaced at regular intervals between 0.5/0.5 and 0.1/0.9, one is assured of testing some bi-allelic markers whose two allele frequencies are reasonably close to the allele frequencies of an unknown bi-allelic disease locus.

Thus, for step 2, within each chromosomal segment, subsets of bi-allelic markers should be identified. Each subset contains only bi-allelic markers having approximately the same allele frequencies. For example, subset A contains only markers whose less common allele has a population frequency of about 0.1. Similarly, subsets B, C, D, and E contain only bi-allelic markers whose less common allele has a frequency of approximately 0.2, 0.3, 0.4, and 0.5, respectively. In other versions of the invention the number of subsets is greater or less than five, and the approximate allele frequency of the less common bi-allele of subsets is other than about 0.1, 0.2, 0.3, 0.4 or 0.5 and is expected to be more than one decimal long since allele frequencies from real populations are rarely round numbers. However, the crucial point is that each subset should contain only bi-allelic markers belonging to one chromosomal segment and the frequency of the less common allele of each subset member should be approximately the same (i.e., the *difference* between the frequencies of the less common allele of any two subset members should not exceed 0.15). Also crucial, as I emphasized above, is that the *group* of subsets for each chromosomal segment represent frequencies near the extremes of 0.5/0.5 and 0.1 /0.9 as well as represent bi-allele frequencies between these two extremes that are approximately evenly spaced as *illustrated* by the group of subsets refereed to above as A, B, C, D and E.

Step 3

In step 2, I described the importance of testing subsets of bi-allelic markers having approximately the same frequencies for their two alleles. Here I further delineate the characteristics of the markers that should be chosen for each subset by noting why it is important that each subset contain more than one bi-allelic marker. Even though a particular bi-allelic marker has allele frequencies that are similar to those of a closely linked bi-allelic disease locus, the marker may not be in strong positive disequilibrium with the disease locus. If disequilibrium is minimal, the marker will not show strong evidence for linkage under the TDT or any other association-based linkage test, *even though the bi-allelic marker and disease locus have similar allele frequencies.*

Hence, it is important that each subset contain multiple bi-allelic markers so that there is increased likelihood that at least one of the markers will be in reasonably strong disequilibrium with a closely linked bi-allelic disease locus. Beyond the cardinal criterion that all bi-allelic markers in a subset have similar allele frequencies, an additional criteria for selecting markers to belong to a subset is that the chosen bi-allelic markers *should not be in extreme positive disequilibrium with each other.*

The reason for this is as follows: According to standard usage, the disequilibrium coefficient ($\delta$) is defined by the equation $\delta = f(AB) - f(A)f(B)$ where $f(A)$ and $f(B)$ may be defined as the frequencies of the less common allele (denoted A and B) of two bi-allelic loci belonging to the same subset and $f(AB)$ is the population frequency of the AB haplotype. Since the two markers belong to the same subset, we may assume that $f(A) = f(B) = q$; hence the maximum positive value of $\delta$ (denoted $\delta_{max}$) is $\delta = q - q^2$. This maximum positive $\delta$ value (i.e. maximum "positive disequilibrium") occurs if every chromosome that carries allele A also carries allele B, and if every chromosome that carries allele not-A also carries allele not-B. Hence, when two bi-allelic markers with similar allele frequencies are in extreme postive disequilibrium with each other (i.e., $\delta$ is approximately equal to $\delta_{max}$), the two loci provide the nearly identical information with respect to their linkage and association with a third polymorphism such as a disease locus. Hence one of the two bi-allelic markers would provide no additional information and its inclusion in the subset would not increase the likelihood of detecting linkage and association to a nearby disease locus.

Therefore, bi-allelic markers belonging to the same chromosomal segment and subset should not only have similar allele frequencies, the $\delta$ value between *each pair* of bi-allelic markers in the same subset should be substantially less than $\delta_{max} = q - q^2$. This assures that every bi-allelic polymorphism belonging to the subset provides much new (i.e. non-redundant) information about linkage and association to any nearby bi-allelic disease locus: thus testing each bi-allelic marker in the subset would increase the likelihood of detecting linkage to a disease locus.

Step4: Test for linkage

To test for (association-based) linkage to a bi-allelic disease locus, each bi-allelic marker in each subset from each chromosomal segment is tested *individually* by using the TDT, AFBAC method or other family-based linkage test. To conduct these tests for a particular marker, members of nuclear families (most especially parents, and any children who manifest disease) are genotyped at the marker being tested and the genotypes are then evaluated according to the TDT, AFBAC method or other family-based linkage/association test (for description of TDT and AFBAC, see Spielman et al, Am J of Human Genetics 52:506-516 (1993) and Thomson, Am J Human Genetics 57:487-498 (1995)). Alternatively, linkage and association is tested for each marker in each subset from each segment by genotyping individuals with disease and related or unrelated normal controls at each marker to be tested.

(End of best mode example)

Further Information

(Step 3 is not essential for the operation or utility of this version of the invention. In this best mode example, the least common allele frequency subrange 0.1 to 0.5 is used. In versions of the invention similar to the best mode, versions of the invention are operable and have utility for any subrange of the least common allele frequency range 0 to 0.5. In addition, rather than genotyping DNA from single individuals in step 4, in some versions of the invention each marker in each subset from each segment is tested for association with disease by evaluating DNA from pooled samples.)
PART 2 - Physical implementation of the above test

Silicon chips or glass slides with arrays of oligonucleotides for testing bi-allelic markers

Companies like Affymetrix[VIII] are using silicon chips or glass slides to genotype DNA from one individual at thousands of bi-allelic markers. Each silicon chip or glass slide is divided into a grid or 2-dimensional matrix that contains thousands of cells. To the surface of each cell is attached multiple copies of a unique oligonucleotide whose sequence is complementary (type (1))to one of the two alleles of a particular bi-allelic marker. Thus, DNA from an individual who carries the allele hybridizes to the cell with substantially greater affinity than does the alternate bi-allele. The degree of hybridization generates a signal and enables the genotype of the individual to be inferred for that particular bi-allelic polymorphism [i.e., the individual is homozygous (++), heterozygous (+-), or homozygous (--)]. In some applications, it is crucial to attach oligonucleotides corresponding to each allele of a bi-allelic polymorphism in adjacent cells so that the relative (i.e. local) intensity of hybridization in the adjacent cells can be compared, thus facilitating inference of the individual's correct genotype (++, +-, or --).
In using this silicon chip or glass slide technology to test for linkage and association, the ideas detailed in PART 1 indicate how the oligonucleotides that are attached to the cells of the silicon chip or glass slide should be chosen. To scan a particular chromosome or chromosomal region for a bi-allelic disease locus, the chromosome or chromosomal region should be subdivided into segments as described in Step 1 above. For each segment, subsets of bi-allelic markers having the properties detailed in PART 1 above should be identified. The DNA of select individuals (see "Test for linkage" - above) should then be assayed at each bi-allelic marker in every chromosomal segment and in every subset of markers belonging to the segment. This would be accomplished by attaching an oligonucleotide corresponding to one of the marker's two alleles to a particular (i.e. known) cell on the silicon chip or slide. To enhance assignment of accurate genotypes, it may also be advisable to attach an oligonucleotide corresponding to the second allele of the bi-allelic marker in an adjacent cell as mentioned in the previous paragraph.

**Industrial Applicability**

Versions of the present invention are useful for locating trait causing genes and polymorphisms such as human disease genes and polymorphisms. Versions of the invention could be used to find the cure for human disease. The making and use of versions of the invention should be clear to a person of skill in the art after reading the description.

Notes:

The reader's attention is directed to the following papers which are open to the public-(1) McGinnis, Ewens & Spielman, Genetic Epidemiology 1995 ; 12(6): 637-40. (2) RE McGinnis Annals of Human Genetics vol 62, pp. 159-179, 1998.

[I] Weighing DNA for Fast Genetic Diagnosis, Science, March 27, 1998, vol. 279, pp. 2044-2045.
[II] Spielman, R.S. and Ewens, W.J. The TDT and Other Fainily-Based Tests for Linkage Disequilibrium and Association , American Journal of Human Genetics, 59: 983-989, 1996.
[III] "Mathematical Theory of Optimization" The New Encyclopedia Britannica, 15th edition, vol. 25, pp. 217-221.
[IV] American Journal of Human Genetics, vol. 57: 439-454, 1995.
[V] American Journal of Human Genetics, vol. 58: 1347-1363, 1996.

[VI] Human Heredity, vol. 44, pp. 225-237, 1994.

[VII] Human Heredity, vol. 46, pp. 226-235, 1996.

[VIII]: Accessing Genetic Information with High-Density DNA Arrays, Mark Chee, et al. Science, vol 274, Oct. 25, 1996 , pp. 610 - 614.

[IX] Large Scale Identification, Mapping, and Genotyping of Single-Nucleotide Polymorphisms in the Human Genome, Wang, et. al., Science, May 15, 1998, vol 280, pp. 1077-1081.

[X] (1)Schuster, H. et al (1995) Nature Genetics, 13(1) : 98 - 100. (2)Gyapay, G. et al (1994) Nature Genetics, 7: 246-339.

[XI] Some versions of oligonucleotide technology and it's uses to obtain genetic information are included in the following papers:

(1) Accessing Genetic Information with High-Density DNA Arrays, Mark Chee, et al. Science, vol 274, Oct. 25, 1996 , pp. 610 - 614.

(2)Genetic analysis of amplified DNA with immobilized sequence- specific oligonucleotide probes, Saiki,et al. Proc Natl Acad Sci USA vol 86, pp.6230-6234.

(3)Allele-specific enzymatic amplification of β-globin genomic DNA for diagnosis of sickle cell anemia, Wu, et al., Proc Natl Acad Sci USA vol 86 pp 2757-2760.

(4)Automated DNA diagnostics using an Elisa-based oligonucleotide ligation assay, Nickerson, et al., Proc Natl Acad Sci USA vol 87, pp. 8923-8927.

(5)Genetic analysis of amplified DNA with immobilized sequence specific oligonucleotide probes, Saiki, et al., Proc Natl Acad Sci USA vol 86 pp 6230 - 6234.

[XII] Padlock Probes:Circularizing Oligonucleotides for Localized DNA Detection, Science, Sept. 30, 1994, vol. 265, pp. 2085-2088.

[XIII] SNP attack on complex traits, Nature Genetics, Nov. 1998, vol. 20 no. 3, pp. 217-218.

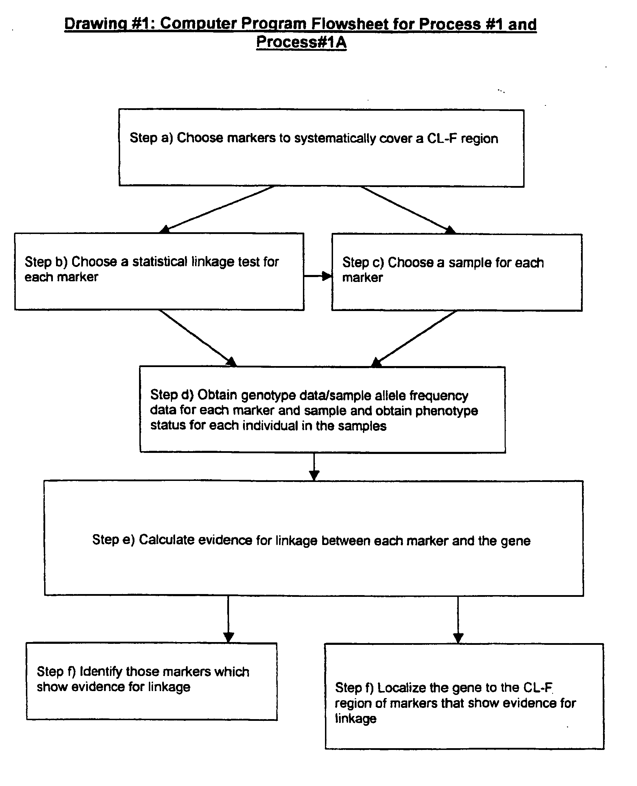

**Claims**

1.  A process for identifying one or more bi-allelic markers linked to a trait-causing polymorphism in a species of creatures, comprising the steps of:

    a) choosing a group of two or more bi-allelic covering markers so that a CL-F region is N covered to within [x, y] by the two or more bi-allelic covering markers, wherein the CL-F region is a collection of one or more points on a CL-F map, the CL-F map is a two-dimensional map like an x-y graph, wherein each point in the region is within the two-dimensional distance [x, y] of N or more of the covering markers and $N \geq 1$;

    i) wherein the x axis on the CL-F map is the chromosomal location (CL) dimension and the y axis of the map is the least common allele frequency (F) dimension, wherein any one bi-allelic polymorphism is viewed as located at a point on the map, wherein the chromosomal location of the one polymorphism is the chromosomal location coordinate of the point and the least common allele frequency of the one polymorphism is the frequency coordinate of the point, wherein chromosomal location coordinates are given in units of centiMorgans or base pairs or an equivalent and wherein frequency coordinates are given in units of least common allele frequency, wherein a two-dimensional distance $[D_x, D_y]$ is defined as the distance between two points, wherein $D_x$ is the absolute difference between the chromosomal location coordinates of the two points and $D_y$ is the absolute difference between the least common allele frequency coordinates of the two points;

    ii) wherein the CL-F region is for a species and a population and the population is a group of individuals as in the field of population genetics;

    iii) wherein the group of covering markers is chosen based on the assumption that the trait-causing polymorphism is located at a point in the CL-F region, wherein y is less than or equal to about 0.2 and wherein x is less than or equal to about 12 cM or about 12 million base pairs or the equivalent thereof;

    iv) wherein the group of two or more covering markers is a marker panel for a linkage study chosen based on using the principle of chromosomal location dimensional closeness of a marker and a nearby polymorphism to achieve linkage between the marker and the nearby polymorphism, wherein the marker panel is specifically selected or specifically chosen based on using the mathematical principle of similarity of covering marker allele frequency and trait-causing polymorphism allele frequency to increase the power of an association-based linkage test to detect evidence for linkage;

v) wherein the process is based on using the increased power of an association-based linkage test to detect linkage when allele frequencies m and p are similar in magnitude, but differ significantly or $\delta$ differs significantly from $\delta_{max}$, wherein each of one or more covering markers that is chosen for possible linkage to the trait-causing polymorphism is chosen based on being a model marker as in an algebraic model, wherein the algebraic model has a model bi-allelic trait-causing polymorphism with trait-causing allele D, wherein the model marker is a bi-allelic marker with alleles A and B, wherein the model marker and the model trait-causing polymorphism are linked, wherein m is the allele frequency of allele A, wherein p is the allele frequency of D, alleles A and D are positively associated and $\delta$ is the coefficient of disequilibrium, wherein $\delta = f(AD) - mp$, wherein f(AD) is the population frequency of the AD haplotype, wherein the model trait-causing polymorphism represents the trait-causing polymorphism, wherein it assumed that $|m - p| \leq y$ and wherein it is assumed that $m/p \geq 2$ or $m/p \leq 0.5$ or $|m - p| \geq 0.1$ or $0 < \delta \leq 1/2\, \delta_{max}$;

vi) wherein each covering marker is a true bi-allelic marker or a bi-allelic marker equivalent (BME), wherein a BME is a mathematical marker that acts mathematically like it is a single true bi-allelic marker, wherein the BME is formed by combining the true alleles or haplotypes from one or more true markers into two groups, and wherein each true marker is an exactly or approximately bi-allelic marker, wherein an exactly bi-allelic marker always occurs in one of two allele forms, wherein an approximately bi-allelic marker has three or more alleles, wherein the approximately bi-allelic marker almost always occurs in one of two allele forms, wherein the two allele forms are a first allele and a second allele, wherein the sum of the allele frequency of the first allele and the allele frequency of the second allele is approximately 1, wherein the approximately bi-allelic marker very rarely occurs as an other allele, wherein the other allele is an allele other than the first or second alleles, wherein the frequency of the first allele and the frequency of the second allele is much greater than the sum of the allele frequencies of all the alleles of the approximately bi-allelic marker that are other than the first or the second alleles;

b) choosing a statistical linkage test based on allelic association for each covering marker;

c) choosing a sample of individuals for each covering marker;

d) obtaining genotype data/sample allele frequency data for each covering marker and the sample chosen for each covering marker, and obtaining phenotype status data for the genetic trait for each individual in the sample chosen for each covering marker, wherein the genotype data/sample allele frequency data is sample allele frequency data or genotype data;

e) calculating evidence for linkage between each covering marker and the trait-causing polymorphism using the statistical linkage test based on allelic association chosen for each covering marker and the genotype data/sample allele frequency data for each covering marker and using the phenotype status data for the genetic trait for each individual in the sample chosen for each covering marker obtained in d); and

f) identifying those covering markers as linked to the trait-causing polymorphism which show evidence for linkage based on the calculations of step e).

2. A process as in claim 1, wherein it is assumed that $m \leq 0.2$.

3. A process as in claims 1-2, wherein the CL-F region is a segment-subrange, and the subrange of the segment-subrange is the subrange 0 to 0.5, whereby the width of the subrange is 0.5, wherein the segment of the segment-subrange is a segment of a chromosome, and wherein the length of the segment is equal to the length of the chromosome, whereby the segment-subrange is a rectangular region, wherein each point in the region is within the subrange and within the segment, wherein [x, y] is [250, 000 bp, 0.1], wherein $N \geq 2$, and wherein N is less than maximal, whereby the number and distribution of known markers in the neighborhood of the CL-F region make it possible for N to be a greater value.

4. A process as in any one of claims 1-3, wherein the sample allele frequency data is obtained from pooled DNA specimens.

5. A process as in any one of claims 1-4, wherein the genotype data/sample allele frequency data is genotype data.

6. A process as in any one of claims 1-5, wherein each covering marker is a true bi-allelic marker and each covering marker is exactly bi-allelic.

7. A process as in any one of claims 1-6, wherein it is assumed that $m/p \geq 2$ or $m/p \leq 0.5$ or $0 < \delta \leq 1/2\, \delta_{max}$.

8. A process as in any one of claims 1-7, wherein it is assumed that $m/p \geq 2$ or $m/p \leq 0.5$.

9. A process as in any one of claims 1-2, and 4-8, wherein the CL-F region is a segment-subrange, and the subrange of the segment-subrange is the subrange 0 to 0.1, wherein the segment of the segment-subrange is a segment of a chromosome, and wherein the length of the segment is equal to the length of the chromosome, whereby the segment-subrange is a rectangular region, wherein each point in the region is within the subrange and within the segment, wherein [x, y] = [250, 000 bp, 0.1], wherein $N \geq 2$, and wherein N is less than maximal, whereby the number and distribution of known markers in the neighborhood of the CL-F region make it possible for N to be a greater value.

10. A process as in any one of claims 1-2, and 4-8, wherein y equals 0.2 and x equals 12 cM or 12 million base pairs of the equivalent thereof.

11. A process as in any one of claims 1-10, wherein there are thousands of covering markers.

12. A process for identifying one or more bi-allelic markers linked to a trait-causing polymorphism in a species of creatures, comprising steps a), b), c), d) and e),
    wherein step a) is choosing a group of two or more bi-allelic covering markers so that a CL-F region is N covered to within [x, y] and $N \geq 1$ as in claim 1, wherein the CL-F region is a CL-F region as in claim 1, wherein the magnitude of the CL-F distance [x, y] is as in claim 1, wherein the group of two or more covering markers is a marker panel as in claim 1, wherein each covering marker is a true bi-allelic marker or a BME as in claim 1,
    wherein each of the steps b), c), d) and e) are as in claim 1, and wherein the process further comprises the step of:

    f) localizing the trait-causing polymorphism to the chromosomal location-least common allele frequency (CL-F) location of one or more markers that show evidence for linkage based on the calculations of step e), wherein the localizing uses a technique or techniques that detects gradients, wherein the detection technique or techniques uses a gradient along the allele frequency dimension, whereby localizing the trait-causing polymorphism to the CL-F location is equivalent to localizing the trait-causing polymorphism to a particular chromosomal location or a particular chromosomal region.

13. A process as in claim 12, wherein each covering marker is a true bi-allelic marker and each covering marker is exactly bi-allelic.

14. A process as in any one of claims 12 or 13, wherein y equals 0.2 and x equals 12 cM or 12 million base pairs or the equivalent thereof.

**Patentansprüche**

1. Verfahren zum Identifizieren eines oder mehrerer biallelischer Marker, die mit einem Merkmals-verursachenden Polymorphismus in einer Spezies von Kreaturen verknüpft sind, welches die Schritte umfasst:

    a) Auswählen einer Gruppe von zwei oder mehr biallelischen bedeckenden Markern, so dass eine CL-F-Region N-fach innerhalb von [x, y] durch die zwei oder mehr biallelischen bedeckenden Marker bedeckt wird, wobei die CL-F-Region eine Sammlung von einem oder mehreren Punkten auf einer CL-F-Karte ist, wobei die CL-F-Karte eine zweidimensionale Karte wie ein x-y-Graph ist, wobei jeder Punkt in der Region sich innerhalb des zweidimensionalen Abstands [x, y] von N oder mehr der bedeckenden Marker befindet und $N \geq 1$;

    i) wobei die x-Achse auf der CL-F-Karte die Dimension der chromosomalen Lage (CL) und die y-Achse der Karte die Dimension der Häufigkeit (F) des am wenigsten häufige Allels ist, wobei ein jeglicher biallelischer Polymorphismus als an einem Punkt auf der Karte lokalisiert angesehen wird, wobei die chromosomale Lage des einen Polymorphismus die Koordinate der chromosomalen Lage des Punkts ist und die Häufigkeit des am wenigstens häufigen Allels des einen Polymorphismus die Häufigkeits-Koordinate des Punkts ist, wobei Koordinaten der chromosomalen Lage in Einheiten von centiMorgan oder Basenpaaren oder einem Äquivalent angegeben sind und wobei Häufigkeits-Koordinaten in Einheiten der Häufigkeit des am wenigsten häufigen Allels angegeben sind, wobei ein zweidimensionaler Abstand $[D_x, D_y]$ als der Abstand zwischen zwei Punkten definiert ist, wobei $D_x$ der absolute Unterschied zwischen den Koordinaten der chromosomalen Lage der zwei Punkte ist und $D_y$ der absolute Unterschied zwischen den Koordinaten der Häufigkeit des am wenigsten häufigen Allels der beiden Punkte ist;
    (ii) wobei die CL-F-Region für eine Spezies und eine Population besteht und die Population eine Gruppe von Individuen wie auf dem Gebiet der Populationsgenetik ist;

(iii) wobei die Gruppe von bedeckenden Markern basierend auf der Annahme ausgewählt wird, dass der Merkmals-verursachende Polymorphismus an einem Punkt in der CL-F-Region lokalisiert ist, in der y weniger als oder gleich etwa 0,2 ist und wobei x weniger als oder gleich etwa 12 cM oder etwa 12 Millionen Basenpaare oder das Äquivalent davon ist;

(iv) wobei die Gruppe von zwei oder mehr bedeckenden Markern ein Marker-Panel für eine Verknüpfungsuntersuchung ist, das basierend auf einer Verwendung des Prinzips der dimensionalen Nähe von chromosomalen Lagen eines Markers und eines nahe gelegenen Polymorphismus ausgewählt ist, um eine Verknüpfung zwischen dem Marker und dem nahe gelegenen Polymorphismus zu erzielen, wobei das Marker-Panel speziell selektiert oder speziell ausgewählt wird basierend auf der Verwendung des mathematischen Prinzips der Ähnlichkeit der Allelhäufigkeit eines bedeckenden Markers und der Allelhäufigkeit eines Merkmals-verursachenden Polymorphismus, um die Leistungsfähigkeit eines Assoziations-basierten Verknüpfungstests zur Erfassung von Anzeichen für eine Verknüpfung zu erhöhen;

(v) wobei das Verfahren auf einer Verwendung der erhöhten Leistungsfähigkeit eines Assoziations-basierten Verknüpfungstests zur Erfassung einer Verknüpfung beruht, wenn Allelhäufigkeiten m und p in der Größe ähnlich sind, sich aber signifikant unterscheiden oder sich $\delta$ signifikant von $\delta_{max}$ unterscheidet, wobei jeder des einen oder der mehreren bedeckenden Marker, der bzw. die für eine mögliche Verknüpfung mit dem Merkmals-verursachenden Polymorphismus ausgewählt wird bzw. werden, darauf basierend ausgewählt wird, ob er ein Modellmarker wie in einem algebraischen Modell ist, wobei das algebraische Modell einen biallelischen Merkmals-verursachenden Modell-Polymorphismus mit einem Merkmals-verursachenden Allel D aufweist, wobei der Modell-Marker ein biallelischer Marker mit Allelen A und B ist, wobei der Modell-Marker und der Merkmals-verursachende Modell-Polymorphismus verknüpft sind, wobei m die Allelhäufigkeit des Allels A ist, wobei p die Allelhäufigkeit von D ist, die Allele A und D positiv assoziiert sind und $\delta$ der Ungleichgewichtskoeffizient ist, wobei $\delta = f(AD) - mp$, worin $f(AD)$ die Populationshäufigkeit des AD-Haplotyps ist, wobei der Merkmals-verursachende Modell-Polymorphismus den Merkmals-verursachenden Polymorphismus repräsentiert, wobei angenommen wird, dass $|m - p| \leq y$ und wobei angenommen wird, dass $m/p \geq 2$ oder $m/p \leq 0,5$ oder $|m - p| \geq 0,1$ oder $0 < \delta \leq 1/2\ \delta_{max}$;

vi) wobei jeder bedeckende Marker ein wahrer biallelischer Marker oder ein Äquivalent eines biallelischen Markers (BME) ist, wobei ein BME ein mathematischer Marker ist, der mathematisch so wirkt, als wäre er ein einzelner wahrer biallelischer Marker, wobei das BME gebildet wird, indem die wahren Allele oder Haplotypen von einem oder mehreren wahren Markern in zwei Gruppen kombiniert werden, und wobei jeder wahre Marker ein exakt oder annähernd biallelischer Marker ist, wobei ein exakt biallelischer Marker stets in einer von zwei Allelformen auftritt, wobei ein annähernd biallelischer Marker drei oder mehr Allele hat, wobei der annähernd biallelische Marker nahezu stets in einer von zwei Allelformen vorkommt, wobei die zwei Allelformen ein erstes Allel und ein zweites Allel sind, wobei die Summe der Allelhäufigkeit des ersten Allels und der Allelhäufigkeit des zweiten Allels ungefähr 1 ist, wobei der nahezu biallelische Marker sehr selten als ein anderes Allel vorkommt, wobei das andere Allel ein anderes Allel als das erste oder das zweite Allel ist, wobei die Häufigkeit des ersten Allels und die Häufigkeit des zweiten Allels viel größer ist als die Summe der Allelhäufigkeiten von allen Allelen des nahezu biallelischen Markers, die andere als das erste oder das zweite Allel sind;

b) Auswählen eines auf einer allelischen Assoziation basierenden statistischen Verknüpfungstests für jeden bedeckenden Marker;

c) Auswählen einer Probe von Individuen für jeden bedeckenden Marker;

d) Gewinnen von Genotyp-Daten/Proben-Allelhäufigkeits-Daten für jeden bedeckenden Marker und die für jeden bedeckenden Marker ausgewählte Probe und Gewinnen von Phänotyp-Status-Daten für das genetische Merkmal für jedes Individuum in der Probe, ausgewählt für jeden bedeckenden Marker, wobei die Genotyp-Daten/Proben-Allelhäufigkeits-Daten Proben-Allelhäufigkeits-Daten oder Genotyp-Daten sind;

e) Berechnen von Anzeichen für eine Verknüpfung zwischen jedem bedeckenden Marker und dem Merkmals-verursachenden Polymorphismus unter Verwendung des auf einer allelischen Assoziation basierenden statistischen Verknüpfungstests, ausgewählt für jeden bedeckenden Marker, und der Genotyp-Daten/Proben-Allelhäufigkeits-Daten für jeden bedeckenden Marker und unter Verwendung der Phänotyp-Statusdaten für das genetische Merkmal für jedes Individuum in der Probe, ausgewählt für jeden bedeckenden Marker, die in d) erhalten worden sind; und

f) Identifizieren von jenen bedeckenden Markern als mit dem Merkmals-verursachenden Polymorphismus verknüpft, welche Anzeichen für eine Verknüpfung basierend auf den Berechnungen von Schritt e) zeigen.

**2.** Verfahren nach Anspruch 1, bei dem angenommen wird, dass $m \leq 0,2$.

**3.** Verfahren nach den Ansprüchen 1-2, bei dem die CL-F-Region ein Segment-Unterbereich ist und der Unterbereich des Segment-Unterbereichs der Unterbereich 0 bis 0,5 ist, wobei die Breite des Unterbereichs 0,5 beträgt, wobei das Segment des Segment-Unterbereichs ein Segment eines Chromosoms ist und wobei die Länge des Segments gleich der Länge des Chromosoms ist, wobei der Segment-Unterbereich eine rechteckige Region ist, wobei jede Punkt in der Region innerhalb des Unterbereichs und innerhalb des Segments liegt, wobei [x, y] [250.000 bp, 0,1] ist, wobei $N \geq 2$ und wobei N kleiner als maximal ist, wodurch die Anzahl und Verteilung von bekannten Markern in der Nachbarschaft der CL-F-Region es möglich machen, dass N ein größerer Wert ist.

**4.** Verfahren nach einem der Ansprüche 1-3, bei dem die Proben-Allelhäufigkeits-Daten ausgehend von gepoolten DNA-Proben erhalten werden.

**5.** Verfahren nach einem der Ansprüche 1-4, bei dem die Genotyp-Daten/Proben-Allelhäufigkeits-Daten Genotyp-Daten sind.

**6.** Verfahren nach einem der Ansprüche 1-5, bei dem jeder bedeckende Marker ein wahrer biallelischer Marker ist und jeder bedeckende Marker exakt biallelisch ist.

**7.** Verfahren nach einem der Ansprüche 1-6, bei dem angenommen wird, dass $m/p \geq 2$ oder $m/p \leq 0,5$ oder $0 < \delta \leq 1/2\ \delta_{max}$ .

**8.** Verfahren nach einem der Ansprüche 1-7, bei dem angenommen wird, dass $m/p \geq 2$ oder $m/p \leq 0,5$.

**9.** Verfahren nach einem der Ansprüche 1-2 und 4-8, bei dem die CL-F-Region ein Segment-Unterbereich ist und der Unterbereich des Segment-Unterbereichs der Unterbereich 0 bis 0,1 ist, wobei das Segment des Segment-Unterbereichs ein Segment eines Chromosoms ist und wobei die Länge des Segments gleich der Länge des Chromosoms ist, wodurch der Segment-Unterbereich eine rechteckige Region ist, wobei jeder Punkt in der Region innerhalb des Unterbereichs und innerhalb des Segments liegt, wobei [x, y] = [250.000 bp, 0,1], wobei $N \geq 2$ und wobei N kleiner als maximal ist, wodurch die Anzahl und Verteilung von bekannten Markern in der Nachbarschaft der CL-F-Region es ermöglichen, dass N ein größerer Wert ist.

**10.** Verfahren nach einem der Ansprüche 1-2 und 4-8, bei dem y gleich 0,2 ist und x gleich 12 cM oder 12 Millionen Basenpaare oder das Äquivalent davon ist.

**11.** Verfahren nach einem der Ansprüche 1-10, bei dem es Tausende von bedeckenden Markern gibt.

**12.** Verfahren zum Identifizieren eines oder mehrerer biallelischer Marker, die mit einem Merkmals-verursachenden Polymorphismus in einer Spezies von Kreaturen verknüpft sind, welches die Schritte a), b), c), d) und e) umfasst, wobei Schritt a) darin besteht, eine Gruppe von zwei oder mehr biallelischen bedeckenden Markern so auszuwählen, dass eine CL-F-Region N-fach innerhalb von [x, y] bedeckt wird und $N \geq 1$, wie in Anspruch 1, wobei die CL-F-Region eine CL-F-Region wie in Anspruch 1 ist, wobei die Größe des CL-F-Abstands [x, y] wie in Anspruch 1 ist, wobei die Gruppe von zwei oder mehr bedeckenden Markern ein Marker-Panel wie in Anspruch 1 ist, wobei jeder bedeckende Marker ein wahrer biallelischer Marker oder ein BME wie in Anspruch 1 ist,
wobei jeder der Schritte b), c), d) und e) wie in Anspruch 1 ist und wobei das Verfahren ferner den Schritt umfasst:

> f) Lokalisieren des Merkmals-verursachenden Polymorphismus an der chromosomalen Position-am wenigsten häufigen Allelhäufigkeits(CL-F)-Position eines oder mehrerer Marker, die Anzeichen für eine Verknüpfung basierend auf den Berechnungen von Schritt e) zeigen, wobei das Lokalisieren eine Technik oder Techniken verwendet, die Gradienten erfasst oder erfassen, wobei die Erfassungstechnik oder -techniken einen Gradienten entlang der Allelhäufigkeits-Dimension verwendet bzw. verwenden, wodurch ein Lokalisieren des Merkmals-verursachenden Polymorphismus an der CL-F-Position äquivalent ist zu einem Lokalisieren des Merkmals-verursachenden Polymorphismus an einer bestimmten chromosomalen Position oder in einer bestimmten chromosomalen Region.

**13.** Verfahren nach Anspruch 12, bei dem jeder bedeckende Marker ein wahrer biallelischer Marker ist und jeder bedeckende Marker exakt biallelisch ist.

**14.** Verfahren nach einem der Ansprüche 12 oder 13, bei dem y gleich 0,2 ist und x gleich 12 cM oder 12 Millionen Basenpaare oder das Äquivalent davon ist.

**Revendications**

1. Procédé d'identification d'un ou de plusieurs marqueurs bialléliques liés à un polymorphisme responsable d'un trait chez une espèce de créa-tures, comprenant les étapes consistant à :

a) choisir un groupe de deux marqueurs couvrants bialléliques ou plus, de façon à ce qu'une région CL-F soit couverte dans les limites [x,y] de N par les deux marqueurs couvrants bialléliques ou plus, dans lequel la région CL-F est un groupe d'un ou de plusieurs points sur une carte CL-F, la carte CL-F étant une carte bidimensionnelle du type graphique x-y, dans lequel chaque point de la région est dans les limites de la distance bidimensionnelle [x,y] de N ou d'un nombre plus important de marqueurs couvrants et $N \geq 1$ ;

i) dans lequel l'axe des x sur la carte CL-F représente la Localisation chromosomique (CL) et l'axe des y de la carte représente la Fréquence allélique la moins courante (F), dans lequel tout polymorphisme biallélique est représenté par un point situé sur la carte, dans lequel la localisation chromosomique dudit polymorphisme correspond aux coordonnées de localisation chromosomique du point et la fréquence allélique la moins courante dudit polymorphisme correspond aux coordonnées de fréquence du point, dans lequel les coordonnées de localisation chromosomique sont indiquées en unités centiMorgan ou paires de bases ou équivalents, et les coordonnées de fréquence sont indiquées en unités de fréquence allélique la moins courante, une distance bidimensionnelle $[D_x, D_y]$ étant définie comme la distance entre deux points, $D_x$ étant la différence absolue entre les coordonnées de localisation chromosomique des deux points et $D_y$ étant la différence absolue entre les coordonnées de fréquence allélique la moins courante des deux points ;

ii) dans lequel la région CL-F est celle d'une espèce et d'une population et la population est un groupe d'individus au sens utilisé dans le domaine de la génétique des populations ;

iii) dans lequel le groupe de marqueurs cou-vrants est choisi en partant de l'hypothèse que le polymorphisme responsable du trait est localisé en un point de la région CL-F, dans lequel y est inférieur ou égal à environ 0,2 et dans lequel x est inférieur ou égal à environ 12 cM ou environ 12 millions de paires de bases ou son équivalent ;

iv) dans lequel le groupe de deux marqueurs couvrants ou plus est un panel de marqueurs pour une étude de liaisons, choisi en fonction du principe de proximité de la dimension Localisation chromosomique d'un marqueur et d'un polymorphisme proche permettant la liaison entre le marqueur et le polymorphisme proche, dans lequel le panel de marqueurs est spécifiquement sélectionné ou spécifiquement choisi selon le principe mathématique de similarité de la fréquence allélique des marqueurs couvrants et de la fréquence allélique du polymorphisme responsable du trait pour augmenter la puissance d'un test de liaison basé sur des associations destiné à mettre en évidence une liaison ;

v) dans lequel le procédé se base sur l'utilisation de la puissance accrue d'un test de liaison basé sur des associations pour détecter une liaison quand les fréquences alléliques m et p sont d'un ordre de grandeur similaire, mais diffèrent signi-ficativement ou que δ diffère significativement de $\delta_{max}$, dans lequel chacun du ou des marqueurs couvrants qui est choisi pour une possible liaison au polymorphisme responsable du trait est choisi à titre de marqueur modèle comme dans un modèle algébrique, dans lequel le modèle algébrique possède un polymorphisme modèle responsable du trait biallélique ayant un allèle responsable du trait D, dans lequel le marqueur modèle est un marqueur biallélique ayant des allèles A et B, dans lequel le marqueur modèle et le polymorphisme modèle responsable du trait sont liés, dans lequel m est la fréquence allélique de l'allèle A, dans lequel p est la fréquence allélique de D, les allèles A et D sont positivement associés et δ est le coefficient de déséquilibre, dans lequel δ = f(AD) - mp, f(AD) étant la fréquence dans la population de l'haplotype AD, dans lequel le polymorphisme modèle responsable du trait représente le polymorphisme responsable du trait, dans lequel on suppose que |m - p| ≤ y et dans lequel on suppose que m/p ≥ 2 ou m/p ≤ 0,5 ou |m - p| ≥ 0,1 ou $0 < \delta \leq \frac{1}{2} \delta_{max}$ ;

vi) dans lequel chaque marqueur couvrant est un véritable marqueur biallélique ou un équivalent de mar-queur biallélique (BME), dans lequel un BME est un marqueur mathématique qui agit mathématiquement comme un véritable marqueur biallélique individuel, dans lequel le BME est formé en combinant les véritables allèles ou haplotypes provenant d'un ou de plusieurs véritables marqueurs en deux groupes, et dans lequel chaque véritable marqueur est un marqueur exactement ou approximativement biallélique, dans lequel un marqueur exactement biallélique apparaît toujours sous l'une des deux formes alléliques, dans lequel un marqueur approximativement biallélique a trois allèles ou plus, dans lequel le marqueur approximativement biallélique apparaît pratiquement toujours sous l'une des deux formes alléliques, dans lequel les deux formes alléliques sont un premier allèle et un second allèle, dans lequel la somme de la fréquence allélique du premier allèle et de la fréquence allélique du second allèle est d'environ 1, dans lequel le marqueur approximativement biallélique apparaît très rarement sous la forme d'un autre allèle, dans lequel ledit autre allèle est un allèle autre que les premiers ou seconds allèles, dans lequel la fréquence du premier allèle et la fréquence du second allèle est très supé-rieure à la somme des fréquences alléliques de tous les allèles du marqueur approximativement biallélique autres que les premiers ou les seconds allèles ;

b) choisir un test de liaison statistique basé sur l'association allélique pour chaque marqueur couvrant ;

c) choisir un échantillon d'individus pour chaque marqueur couvrant ;

d) obtenir les données génotypiques/données de fréquences alléliques de l'échantillon pour chaque marqueur couvrant et l'échantillon choisi pour chaque marqueur couvrant, et obtenir les données phénotypiques de chaque trait génétique de chaque individu de l'échantillon choisi pour chaque marqueur couvrant, dans lequel les données génotypiques/données de fréquences alléliques de l'échantillon sont les données de fréquences alléliques de l'échantillon ou les données génotypiques ;

e) prouver par calculs la liaison entre chaque marqueur couvrant et le polymorphisme responsable du trait en utilisant le test de liaison statistique basé sur l'association allélique choisie pour chaque marqueur couvrant et les données génotypiques/données de fréquences alléliques de l'échantillon pour chaque mar-queur couvrant et en utilisant les données phénotypiques du trait génétique de chaque individu de l'échantillon choisi pour chaque marqueur couvrant obtenues en d) ; et

f) identifier les marqueurs couvrants liés au polymorphisme responsable du trait qui prouvent la liaison selon les calculs de l'étape e).

**2.** Procédé selon la revendication 1, dans lequel on suppose que m ≤ 0,2.

**3.** Procédé selon les revendications 1-2, dans lequel la région CL-F est une sous-plage de segment, et la sous-plage de la sous-plage de segment est la sous-plage de 0 à 0,5, la largeur de la sous-plage étant de 0,5, dans lequel le segment de la sous-plage de segment est un segment de chromosome, et dans lequel la longueur du segment est égale à la longueur du chromosome, la sous-plage de segment étant une région rectangulaire, dans lequel chaque point présent dans la région s'inscrit dans la sous-plage et dans le segment, dans lequel [x,y] est [250 000 pb, 0,1], dans lequel N ≥ 2, et dans lequel N est inférieur au maximum, le nombre et la distribution des marqueurs connus au voisinage de la région CL-F permettant de conférer une plus grande valeur à N.

**4.** Procédé selon l'une quelconque des revendications 1-3, dans lequel les données de fréquences alléliques de l'échantillon s'obtiennent à partir de spécimens d'ADN regroupés en pool.

**5.** Procédé selon l'une quelconque des revendications 1-4, dans lequel les données génotypiques/ données de fréquences alléliques de l'échantillon sont les données génotypiques.

**6.** Procédé selon l'une quelconque des revendications 1-5, dans lequel chaque marqueur couvrant est un véritable marqueur biallélique et chaque marqueur couvrant est exactement biallélique.

**7.** Procédé selon l'une quelconque des revendications 1-6, dans lequel on suppose que m/p ≥ 2 ou que m/p ≤ 0,5 ou $0 < \delta \leq \frac{1}{2}\, \delta_{max}$.

**8.** Procédé selon l'une quelconque des revendications 1-7, dans lequel on suppose que m/p ≥ 2 ou que m/p ≤ 0,5.

**9.** Procédé selon l'une quelconque des revendications 1-2, et 4-8, dans lequel la région CL-F est une sous-plage de segment, et la sous-plage de la sous-plage de segment est la sous-plage de 0 à 0,1, dans lequel le segment de la sous-plage de segment est un segment de chromosome, et dans lequel la longueur du segment est égale à la longueur du chromosome, la sous-plage de segment étant une région rectangulaire, dans lequel chaque point présent dans la région s'inscrit dans la sous-plage et dans le segment, dans lequel [x,y] est [250 000 pb, 0,1], dans lequel N ≥ 2, et dans lequel N est inférieur au maximum, le nombre et la distribution des marqueurs connus au voisinage de la région CL-F permettant de conférer une plus grande valeur à N.

**10.** Procédé selon l'une quelconque des revendications 1-2, et 4-8, dans lequel y est égal à 0,2 et x est égal à 12 cM ou 12 millions de paires de bases ou son équivalent.

**11.** Procédé selon l'une quelconque des revendications 1-10, dans lequel il y a des milliers de marqueurs couvrants.

**12.** Procédé d'identification d'un ou de plusieurs marqueurs bialléliques liés à un polymorphisme responsable d'un trait chez une espèce de créatures, comprenant les étapes a), b), c), d), et e),

dans lequel l'étape a) consiste à choisir un groupe de deux marqueurs couvrants bialléliques ou plus de façon à ce qu'une région CL-F soit couverte dans les limites [x,y] de N et N $\geq$ 1 selon la revendication 1, dans lequel la région CL-F est une région CL-F selon la revendication 1, dans lequel l'ordre de grandeur de la distance CL-F [x, y] est selon la revendication 1, dans lequel le groupe de deux marqueurs couvrants ou plus est un panel de marqueurs selon la revendication 1, dans lequel chaque marqueur couvrant est un véritable marqueur biallélique ou un BME selon la revendication 1,

dans lequel chacune des étapes b), c), d) et e) est selon la revendication 1, et dans lequel le procédé comprend en outre l'étape consistant à :

f) localiser le polymorphisme responsable du trait en fonction de la localisation chromosomique-localisation de la fréquence allélique la moins courante (CL-F) d'un ou de plusieurs marqueurs qui sont liés d'après les calculs de l'étape e), dans lequel la localisation utilise une technique ou des techniques qui détecte(nt) des gradients, dans lequel la technique ou les techniques de détection uti-lise(nt) un gradient le long de la dimension fréquence allélique de façon à ce que la localisation du polymorphisme responsable du trait en fonction de la localisation CL-F revienne à localiser le polymorphisme responsable du trait sur une position chromosomique particulière ou une région chromosomique particulière.

13. Procédé selon la revendication 12, dans lequel chaque marqueur couvrant est un véritable marqueur biallélique et chaque marqueur couvrant est exactement biallélique.

14. Procédé selon l'une quelconque des revendications 12 ou 13, dans lequel y est égal à 0,2 et x est égal à 12 cM ou 12 millions de paires de bases ou son équivalent.

## Drawing #1: Computer Program Flowsheet for Process #1 and Process#1A

Step a) Choose markers to systematically cover a CL-F region

Step b) Choose a statistical linkage test for each marker

Step c) Choose a sample for each marker

Step d) Obtain genotype data/sample allele frequency data for each marker and sample and obtain phenotype status for each individual in the samples

Step e) Calculate evidence for linkage between each marker and the gene

Step f) Identify those markers which show evidence for linkage

Step f) Localize the gene to the CL-F region of markers that show evidence for linkage

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0892068 A1
- WO 0028080 A2
- US 5667976 A

- WO 9820165 A
- WO 9807887 A
- US 5552270 A

### Non-patent literature cited in the description

- Linked polymorphic DNA markers in the prediction of X-linked muscular dystrophy. **Lindlöf.** Annals of Human Genetics. October 1987, vol. 51, 317-28
- **Ramsay.** Haplotype analysis to determine the position of a mutation among closely linked DNA markers. *Human Molecular Genetics,* July 1993, vol. 2 (7), 1007-14
- **Devlin B ; Risch N.** A comparison of linkage disequilibrium measures for fine-scale mapping. *Genomics,* 20 September 1995, vol. 29 (2), 311-22
- **Collins ; Morton.** Mapping a disease locus by allelic association. *Proceedings of the National Academy of Sciences of the U S A.,* 17 February 1998, vol. 95 (4), 1741-5
- **Chee.** Accessing genetic information with high-density DNA arrays. *Science,* 25 October 1996, vol. 274 (5287), 610-4
- **Wang.** Large-scale identification, mapping, and genotyping of single-nucleotide polymorphisms in the human genome. *Science,* 15 May 1998, vol. 280 (5366), 1077-82
- **Yershov.** DNA analysis and diagnostics on oligonucleotide microchips. *Proceedings of the National Academy of Sciences of the U S A.,* 14 May 1996, vol. 93 (10), 4913-8
- **Guo.** Direct fluorescence analysis of genetic polymorphisms by hybridization with oligonucleotide arrays on glass supports. *Nucleic Acids Res.,* 11 December 1994, vol. 22 (24), 5456-65
- **Drobyshev.** Sequence analysis by hybridization with oligonucleotide microchip: identification of beta-thalassemia mutations. *Gene,* 25 March 1997, vol. 188 (1), 45-52
- **Reed.** Chromosome-specific microsatellite sets for fluorescence-based, semi-automated genome mapping. *Nature Genetics,* July 1994, vol. 7, 390-395
- **Levinson.** Genome Scan of Schizophrenia. *Am J Psychiatry,* June 1998, vol. 155, 741-750
- **Kruglyak et al.** Linkage Thresholds for Two-stage Genome Scans. *Am J Hum Genet,* 1998, vol. 62, 994-996

- **Kruglyak.** The use of a genetic map of biallelic markers in linkage studies. *Nature Genetics,* September 1997, vol. 17, 21-24
- **Kruglyak et al.** Complete Multipoint Sib-Pair Analysis of Qualitative and Quantitative Traits. *Am J Hum Genet,* 1995, vol. 57, 439-454
- **Kruglyak et al.** Parametric and Nonparametric Linkage Analysis. *Am J Hum Genet,* 1996, vol. 58, 1347-1363
- **Spielman, R.S. ; McGinnis, R.E. ; Ewens, W.J.** Transmission Test for Linkage Disequilibrium: The Insulin Gene Region and Insulin-dependent Diabetes Mellitus(IDDM). *Am J Hum Genet,* 1993, vol. 52, 506-516
- **Risch, N. ; Merikangas, K.** The Future of Genetic Studies of Complex Human Diseases. *Science,* 13 September 1996, vol. 273, 1516-1517
- **Muller-Myshok, B. ; Abel, L.** Technical Comments: The Future of Complex Diseases. *Science,* 28 February 1997, vol. 275, 1328-1329
- **Risch, N. ; Merikangas, K.** Technical Comments: The Future of Complex Diseases. *Science,* 28 February 1997, vol. 275, 1330
- Hidden Linkage: Comparison of the affected sib pair (ASP) test and transmission disequilibrium test (TDT). **MC Ginnis R.E.** Annals of Human Genetics. 1998, vol. 62, 159-179
- Haplotype relative risks: an easy reliable way to construct a proper control sample for risk calculations. **Falk CT ; Rubenstein P.** Annals of Human Genetics. 1987, vol. 51, 227-233
- **Bell GI ; Horita S ; Karam JH.** A polymorphic locus near the human insulin gene is associated with insulin-dependent diabetes mellitus. *Diabetes.,* 1984, vol. 33, 176-183
- **McGinnis ; Ewens ; Spielman.** *Genetic Epidemiology,* 1995, vol. 12 (6), 637-40
- **RE McGinnis.** Annals of Human Genetics. 1998, vol. 62, 159-179
- **Spielman et al.** *Am J of Human Genetics,* 1993, vol. 52, 506-516

- **Thomson.** *Am J Human Genetics,* 1995, vol. 57, 487-498
- Weighing DNA for Fast Genetic Diagnosis. *Science,* 27 March 1998, vol. 279, 2044-2045
- **Spielman, R.S. ; Ewens, W.J.** The TDT and Other Fainily-Based Tests for Linkage Disequilibrium and Association. *American Journal of Human Genetics,* 1996, vol. 59, 983-989
- Mathematical Theory of Optimization. The New Encyclopedia Britannica. vol. 25, 217-221
- *American Journal of Human Genetics,* 1995, vol. 57, 439-454
- *American Journal of Human Genetics,* 1996, vol. 58, 1347-1363
- *Human Heredity,* 1994, vol. 44, 225-237
- *Human Heredity,* 1996, vol. 46, 226-235
- **Mark Chee et al.** Accessing Genetic Information with High-Density DNA Arrays. *Science,* 25 October 1996, vol. 274, 610-614
- **Wang.** Large Scale Identification, Mapping, and Genotyping of Single-Nucleotide Polymorphisms in the Human Genome. *Science,* 15 May 1998, vol. 280, 1077-1081
- **Schuster, H. et al.** *Nature Genetics,* 1995, vol. 13 (1), 98-100
- **Gyapay, G. et al.** *Nature Genetics,* 1994, vol. 7, 246-339
- **Saiki.** Genetic analysis of amplified DNA with immobilized sequence- specific oligonucleotide probes. *Proc Natl Acad Sci USA,* vol. 86, 6230-6234
- **Wu et al.** Allele-specific enzymatic amplification of β-globin genomic DNA for diagnosis of sickle cell anemia. *Proc Natl Acad Sci USA,* vol. 86, 2757-2760
- **Nickerson et al.** Automated DNA diagnostics using an Elisa-based oligonucleotide ligation assay. *Proc Natl Acad Sci USA,* vol. 87, 8923-8927
- **Saiki et al.** Genetic analysis of amplified DNA with immobilized sequence specific oligonucleotide probes. *Proc Natl Acad Sci USA,* vol. 86, 6230-6234
- Padlock Probes:Circularizing Oligonucleotides for Localized DNA Detection. *Science,* 30 September 1994, vol. 265, 2085-2088
- SNP attack on complex traits. *Nature Genetics,* November 1998, vol. 20 (3), 217-218